# EUROPEAN PATENT SPECIFICATION

(11) **EP 2 099 776 B1**
(45) Date of publication and mention of the grant of the patent: **07.10.2015**
(21) Application number: 07848478.9
(22) Date of filing: 10.12.2007
(51) Int. Cl.: C07D 257/02, A61K 31/395, A61P 35/00

(54) **CHELATING AGENT**
CHELATBILDNER
AGENT CHÉLATANT

(30) Priority: 08.12.2006 GB 0624587
(43) Date of publication of application: 16.09.2009
(73) Proprietor: Technische Universität München, 91675 München (DE)
(72) Inventor: KNÖR, Sebastian, 84543 Winhöring/Untertau (DE); MODLINGER, Armin, 01917 Kamenz (DE); WESTER, Hans-Jürgen, 85304 Illmmünster (DE); KESSLER, Horst, 85748 Garching (DE)
(74) Representative: Jones, Nicholas Andrew
(86) International application number: PCT/GB2007/004733
(87) International publication number: WO 2008/068516

(56) References cited:
- WO-A-2004/082722
- WO-A-2006/110745
- US-A- 5 652 361
- US-A1- 2006 155 120
- KNÖR, S.; ET AL.: "Synthesis of novel 1,4,7,10-tetraazacyclodecane-1,4,7,10-tetr aacetic acid (DOTA) derivatives for chemoselective attachment to unprotected polyfunctionalized compounds" CHEMISTRY - A EURPEAN JOURNAL, vol. 13, 2007, pages 6082-6090, XP002472079

## Description

The present invention relates to chelating agents. In particular, it relates to a series of bifunctional chelating agents for selective attachment to targeting molecules.

For non-covalent binding of radionuclides, bifunctional chelating agents (BFCAs) are used to connect radioactive markers and a targeting molecule. Among these BFCAs, the 1,4,7,10-tetraazacyclododecane-1,4,7,10-tetraacetic acid (DOTA) is a well-studied macrocyclic complex ligand which, in contrast to diethylenetriaminepentaacetic acid (DTPA),¹ has been shown to form extremely stable complexes of both divalent and trivalent metals.^{2,3} Radiopharmaceuticals containing this ligand as metal chelator have found widespread use in therapy and diagnostic imaging.^{4,5}

DOTA-peptides are generally synthesized either in solution⁶ or on solid support attaching the DOTA residue to a free amine of the resin bound peptide using unprotected DOTA,¹ or more conveniently, protected DOTA derivatives to overcome side reactions by polyactivation of the four carboxylic groups of DOTA. Therefore, a number of DOTA-derivatives were developed allowing selective formation of monoconjugates. For example the triprotected and commercially available DOTA-tris(*tert*-butyl) ester⁷ and the corresponding benzyl protected analogues DOTA-tris(benzyl) ester,⁸ the isothiocyanate functionalized *p*-NCS-Bz-DOTA⁹ as well as the DOTAGA(*t*Bu)₄¹⁰ which contains an additional unprotected carboxylic group are widely used BFCAs. In other approaches, derivatized amino acids containing a DOTA moiety in the side chain were used¹¹ or the DOTA moiety was synthesized stepwise on the *N*-terminus of a resin bound peptide.¹² The main limitation of all these methods is the attachment of the DOTA residue in an electrophilic manner. This procedure tolerates no other *N-* or *S*-nucleophilic groups for a selective reaction.

Chemoselective approaches allowing a site-selective functionalization of polyfunctionalized compounds remain in demand. However, such a method would be a powerful tool for the synthesis of DOTA-linked radiopharmaceuticals; enabling new synthetic strategies and the synthesis of complex structures such as our recently developed multimeric aminooxy-functionalized RGD-derivatives,¹³ which has been successfully ¹⁸F-labeled using 4-[¹⁸F]fluorobenzaldehyde.¹⁴ In a recent publication, Hovinen reported the synthesis of an aminooxy-functionalized chelate by derivatization of the tris *tert-butyl* ester of DOTA which was conjugated with naltrexone and 2-deoxy-D-ribose.¹⁵ However, the reported procedure requires expensive starting material and the applicability to polyfunctionalized compounds like peptides remains unclear.

The present invention provides a compound of the formula: 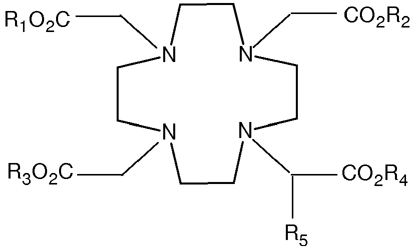 wherein R¹ is selected from H, methyl, ethyl, carboxyl protecting groups and a sugar moiety, R² and R³ are independently selected from H, methyl, ethyl and carboxyl protecting groups, R⁴ is selected from H, methyl, ethyl, a sugar moiety, and carboxyl protecting groups, and R⁵ is an aryl or heteroaryl group substituted with a keto group or a functional group suitable for participating in a cycloaddition reaction and selected from an alkyne group or an azide group, wherein the carboxyl protecting groups, when present, are selected from benzyl, fluorenylmethyl and t-butyl.

Preferably, R⁵ is a 5-9-membered aryl or heteroaryl group comprising one or two rings. More preferably, R⁵ is a 6-membered aryl or heteroaryl group. Even more preferably, R⁵ is a phenyl group. Most preferably, the phenyl group is para-substituted with the keto group or the functional group suitable for participating in a cycloaddition reaction, as described above.

Preferably, the keto group is a methylketone. The advantage of a keto group is that it gives the compound long term stability as it is not a highly reactive group. This allows it to be stored for a long period of time. Highly reactive groups present problems for trying to store compounds for a significant length of time. Further, keto groups allow chemoselective attachment to polyfunctionalized compounds like peptides. Another advantage, is that keto groups do not require additional protection during the synthesis process.

The functional group suitable for participating in a cycloaddition reaction is an alkyne group or an azide group. Preferably, the alkyne group is an ethinyl group.

In one embodiment, R¹, R² and R³ are independently selected from H and carboxyl protecting groups, and R⁴ is selected from H, methyl, ethyl and carboxyl protecting groups. Preferably, R¹, R² and R³ are the same or alternative carboxyl protecting groups. Preferably, R⁴ is H or methyl. More preferably, R¹, R² and R³ are t-butyl and R⁴ is H or methyl.

If carboxyl protecting groups are present, they are selected from benzyl, fluorenylmethyl and t-butyl.

In one embodiment, either R¹ or R⁴ is a sugar moiety. Preferably, R¹ and R⁴ are both sugar moieties. When R⁴ and/or R¹ are sugar moieties, the compound is preferably used to complex Gallium-68. The advantage of attaching sugar moieties to the compound is that they improve the pharmacokinetic properties of the compound.

In another aspect of the invention, there is provided a compound of the formula: 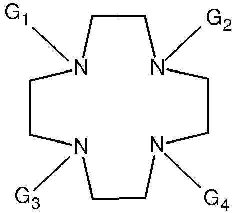 wherein from two to four of the groups G¹ to G⁴ are CH(CO₂R⁴)-R⁵ and any remaining groups G¹ to G⁴ being CH₂CO₂R¹, wherein R⁴ is selected from H, methyl, ethyl and carboxyl protecting groups, R⁵ is as defined above and R¹ is as defined above, wherein the carboxyl protecting groups, when present, are selected from benzyl, fluorenylmethyl and t-butyl. This allows the compound to be attached to multiple targeting molecules, one at each R⁵ group.

The present invention also provides a conjugate comprising a compound as described above and a derivatised targeting molecule, wherein, when R⁵ is substituted with a keto group, the targeting molecule is derivatised to contain a complementary aminooxy moiety, and the compound and targeting molecule are joined by an oxime linkage and, when R⁵ is substituted with a functional group suitable for participating in a cycloaddition reaction and selected from an alkyne group or an azide group, the targeting molecule is derivatised to contain a complementary group for the cycloaddition reaction and the compound and targeting molecule are joined by means of a heterocyclic product of the cycloaddition reaction.

In one embodiment of the conjugate, the compound and targeting molecule are joined by means of a 1,2,3-triazole group.

The present invention also provides a chelate comprising a radionuclide complexed with the compound described above or the conjugate described above. Preferably, the radionuclide is selected from Actinium-225, Bismuth-212, Bismuth-213, Lead-203, Copper-64, Copper-67, Gallium-66, Gallium-67, Gallium-68, Lutetium-177, Indium-111, Indium-113, Yttrium-86 and Yttrium-90, Dysprosium 162, Dysprosium 165, Dysprosium 167, Holmium-166, Praseodymium-142, Praseodymium-143, Promethium-149, and Terbium-149.

The present invention also provides a method of synthesis of a compound according to the invention, the synthesis comprising: the reaction of the di-substituted aryl or heteroaryl L¹-CH(CO₂R⁴)- R⁵-X with cyclen, wherein R⁴ and R⁵ have the same meaning as above, L¹ is a leaving group and X is a keto group or a functional group suitable for participation in a cycloaddition reaction and selected from an alkyne group or an azide group, or a protected form of such a functional group; and the alkylation of the other nitrogen atoms of the cyclen using L₂CH₂CO₂R, wherein R is R¹, R² or R³ as defined above and L² is a leaving group.

The advantage of using cyclen as a starting material is that it is relatively cheap compared to other compounds, for example, the highly expensive DOTA tris *tert-*butyl ester.

The method of synthesis can involve reacting further R⁵ containing groups with cyclen, wherein between two and four of the nitrogen atoms of cyclen are reacted with L¹-CH(CO₂R⁴)-R⁵-X, wherein R⁴ is selected from H, methyl, ethyl and carboxyl protecting groups and R⁵ has the same meaning as above and wherein any remaining nitrogen atoms of the cyclen are alkylated using L²CH₂CO₂R, wherein R is, R² or R³ as defined above.

The invention also provides a method of synthesising the conjugate of the invention by reacting together the compound of the invention and the derivatised targeting molecule prior to the optional complexation of the conjugate with a radionuclide to form a chelate. An advantage of reacting together the compound and the derivatised targeting molecule prior to the complexation of the conjugate with a radionuclide is that the chelating agent may first be further manipulated (e.g. purified and formulated for targeted administration) without the precautions necessary for radionuclide manipulation. Further, the conjugate can be mass produced at a central location before being transported to different locations for use. Another advantage is that the conditions used for complexing the compound to a radionuclide may be quite harsh so that the final conjugate is formed before these harsh conditions are used which could otherwise affect the compound.

In one embodiment of the method, the compound and targeting molecule are joined together by a cycloaddition reaction in the presence of a transition metal catalyst. Preferably, the metal catalyst is based on Cu or Rh.

The invention also provides a chelate as defined above for use in therapy or diagnosis.

Furthermore, the invention provides a chelate as defined above for use for the diagnosis and/or treatment of hyperproliferative and/or neoplastic conditions.

Preferably, the condition in the above uses is cancer. Preferably, the cancer is hormone responsive.

The invention provides the use of a compound according to the invention in the synthesis of a conjugate according to the invention.

Further, the invention provides the use of a conjugate according to the invention in the synthesis of a chelate according to the invention.

The invention also provides a conjugate or a chelate as defined above wherein the targeting molecule is a peptide.

The invention provides a method of dechelating a metal catalyst from a bifunctional chelating agent following the metal catalysed conjugation of the bifunctional chelating agent to a targeting molecule having one or more disulfide bridges, the method comprising the removal of the metal ions using sodium sulfide, followed by treatment with NH₃ and a solvent comprising acetonitrile and water to restore the disulfide bridges. Preferably, the bifunctional chelating agent is the compound of the invention.

In one embodiment, the conjugation reaction involves a cycloaddition reaction.

Preferably, the metal catalyst is based on Cu or Rh.

Finally, the invention provides a conjugate comprising a compound with multiple R⁵ groups, as described above, and two or more targeting molecules joined to the compound through the R⁵ groups.

The invention will now be described in more detail by way of example only with reference to the accompanying figures in which:
Figure 1 shows the structures of 4-acetylphenyl-DOTA-derivatives **1** and **2** and ethinyl-DOTA-derivative **3**;
Figure 2 shows a HPLC trace of crude DOTA conjugate **19** obtained by oxime ligation;
Figure 3 shows a HPLC trace of crude DOTA conjugate **24** obtained after 1,3-dipolar cycloaddition and subsequent deprotection (step 2, Scheme 5);
Figure 4 shows the biodistribution of [⁶⁸Ga]**19** in AR42J tumor bearing nude mice 30 and 60 min p.i. (n = 5; n = 3 for the competition study). Data are given in % injected dose per gram tissue (%iD/g) and are means ± SD;
Figure 5 shows the tumor to non-tumor ratios found for [68Ga]**19** in AR42J tumor bearing nude mice 30 and 60 min p.i. (n = 5). Data are means ± SD;

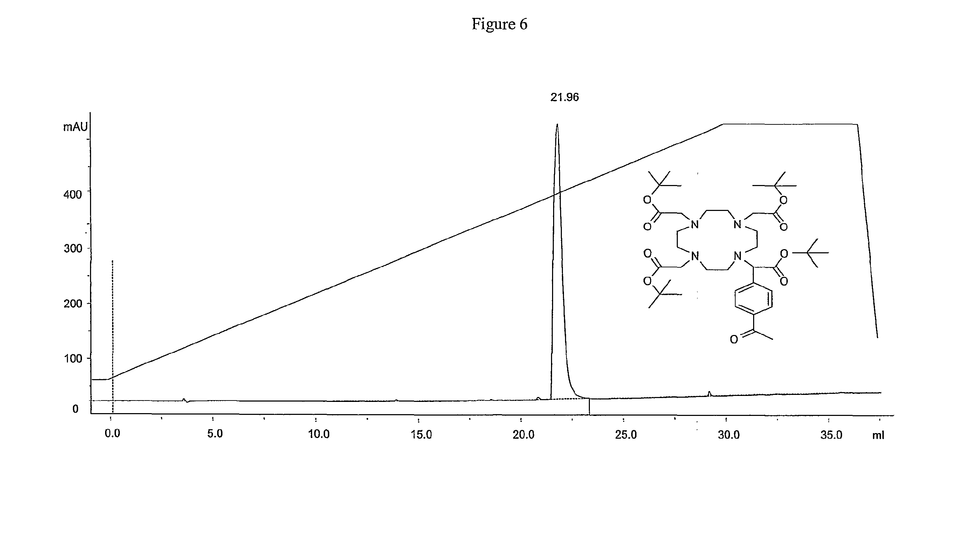

Figure 6 shows the HPLC trace of tert-butyl 2-[1-(1,4,7,10-tetraazacyclododecane)-4,7,10-tris(*tert*-butylacetate)]-(4-acetylphenyl)acetate (**1**). Gradient: 10→80%; 30 min;

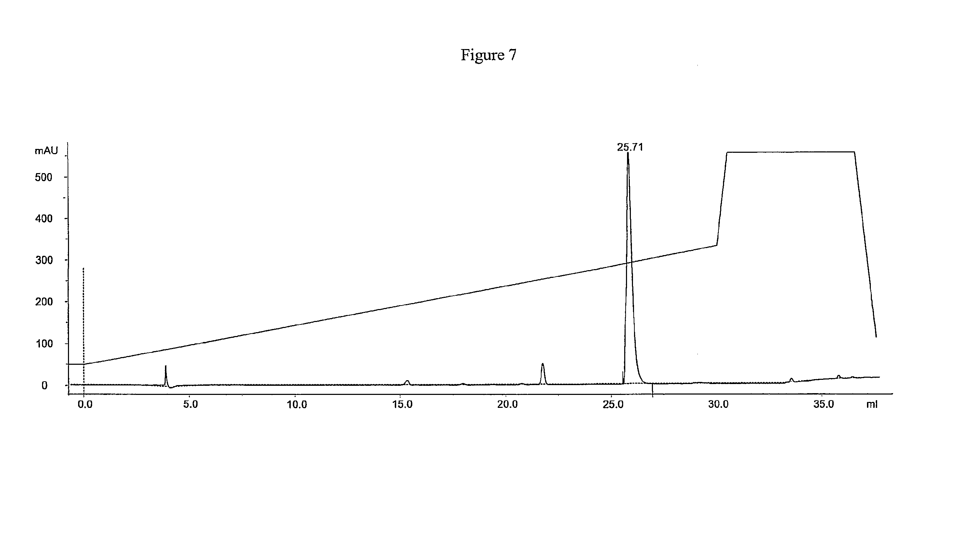

Figure 7 shows the HPLC trace of 2-[1-(1,4,7,10-tetraazacyclododecane)-4,7,10-tris(*tert*-butylacetate)]-(4-acetylphenyl) acetic acid (**2**). Gradient: 10→60%; 30 min;

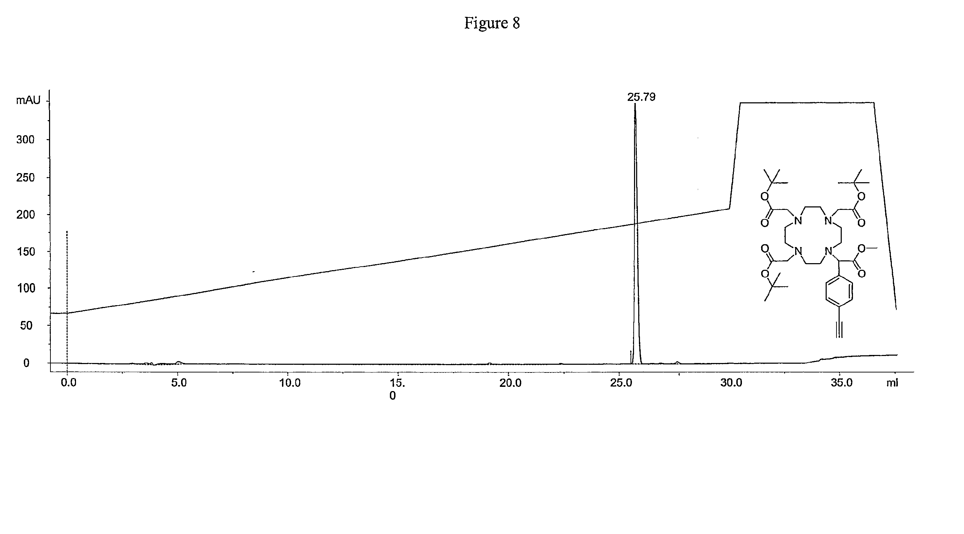

Figure 8 shows the HPLC trace of (*R*/*S*)-methyl 2-[1-(1,4,7,10-tetraazacyclododecane)-4,7,10-tris(*tert*-butylacetate)]-2-(4-ethynyl)phenyl)acetate (**3**). Gradient: 10→100%; 30 min;

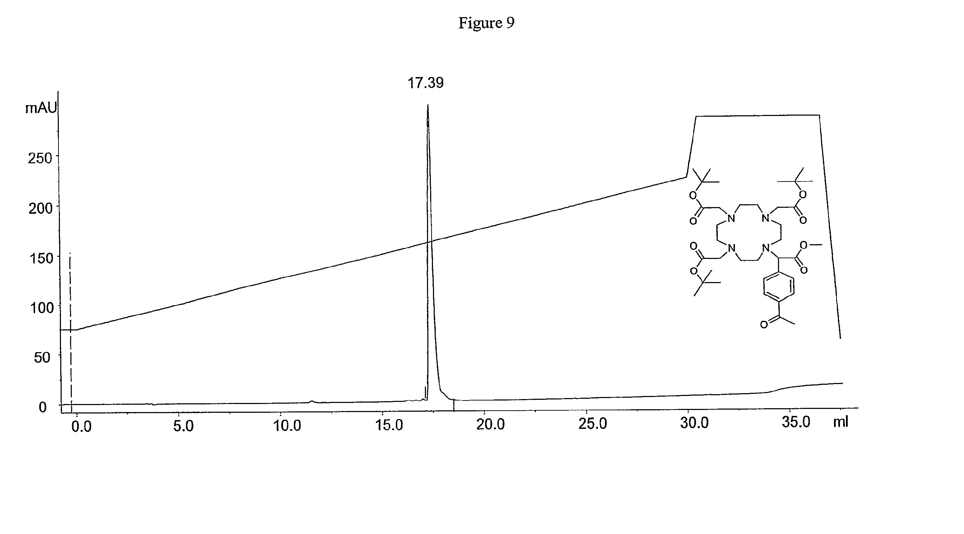

Figure 9 shows the HPLC trace of methyl 2-[1-(1,4,7,10-tetraazacyclododecane)-4,7,10-tris(*tert*-butylacetate)]-(4-acetylphenyl)acetate (**11**). Gradient: 10→80%; 30 min;

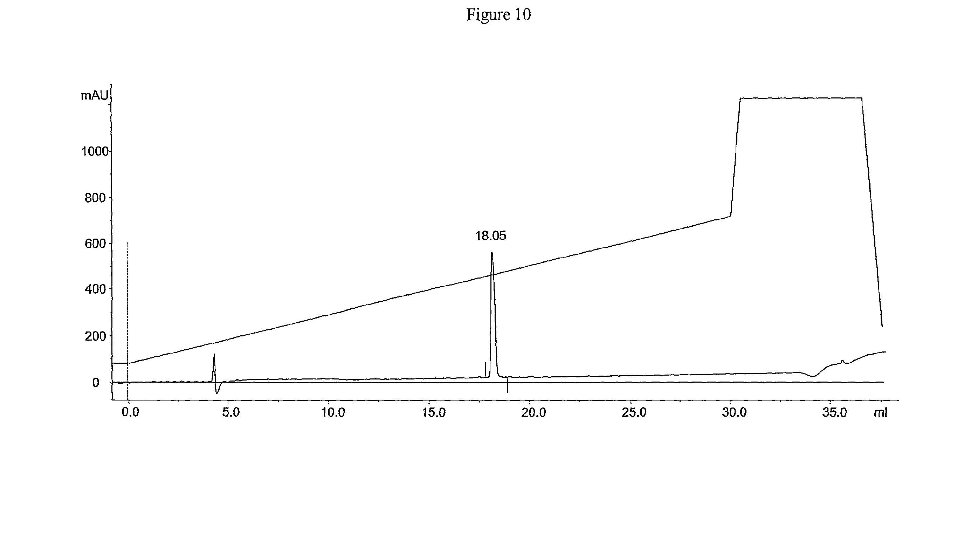

Figure 10 shows the HPLC trace of DOTA-Tyr³-octreotate derivative **19**. Gradient: 10→60%; 30 min;

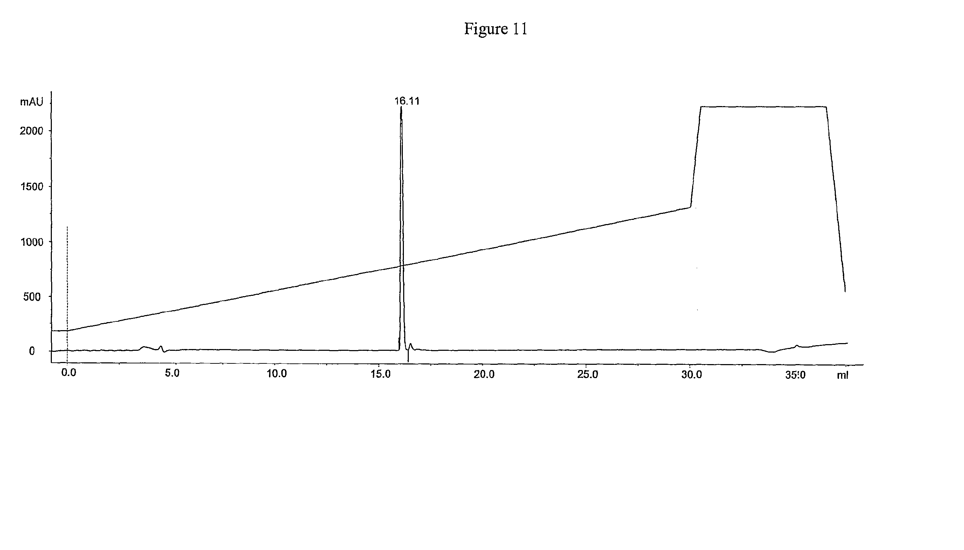

Figure 11 shows the HPLC trace of DOTA-Tyr³-octreotate derivative **24**. Gradient: 10→60%; 60 min;

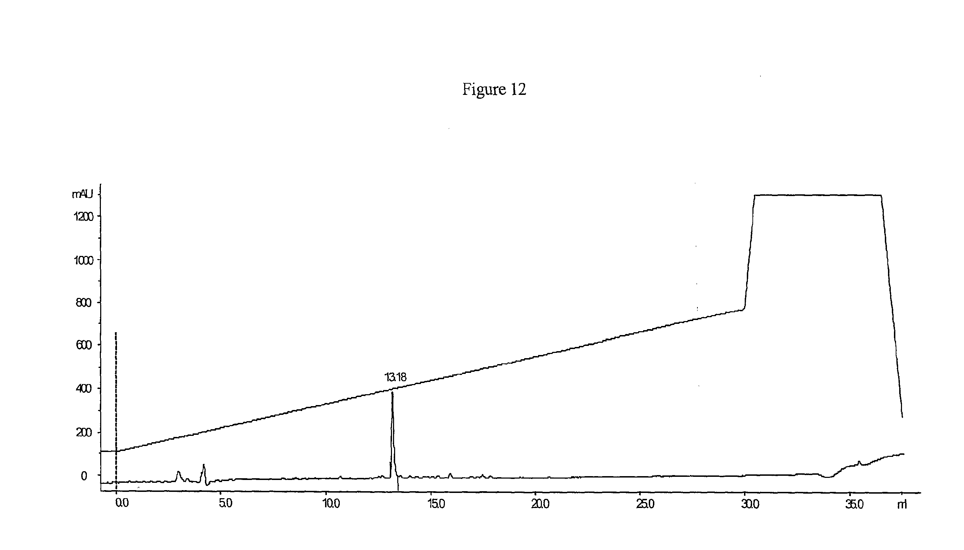

Figure 12 shows the HPLC trace of crude DOTA conjugate **26** obtained by 1,3-dipolar cycloaddition. Gradient: 10→60%; 60 min;

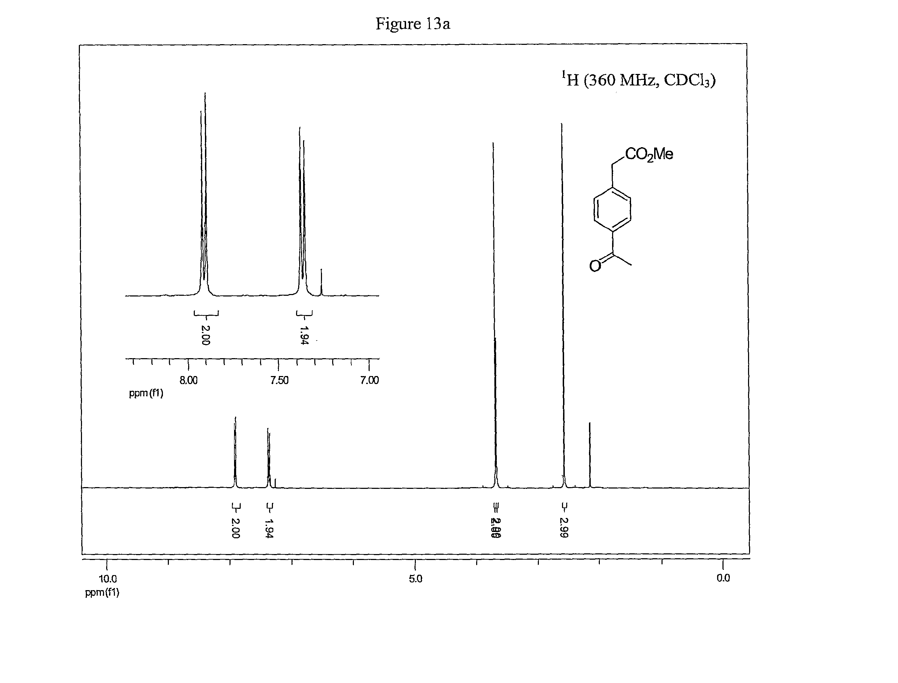

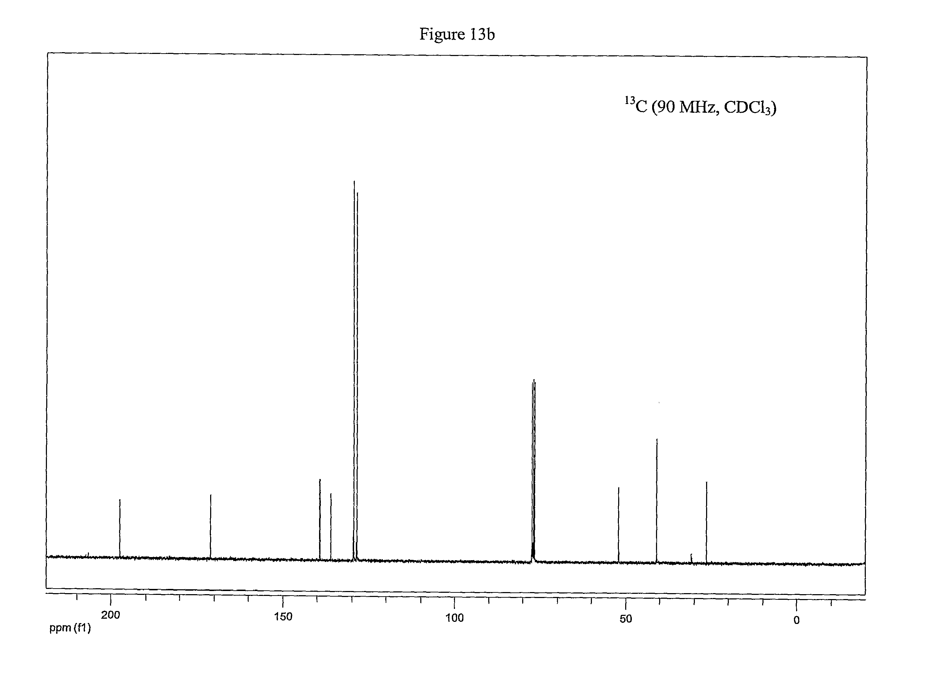

Figure 13 shows the NMR spectra of *tert*-butyl 2-(4-acetylphenyl)acetate (**5**);

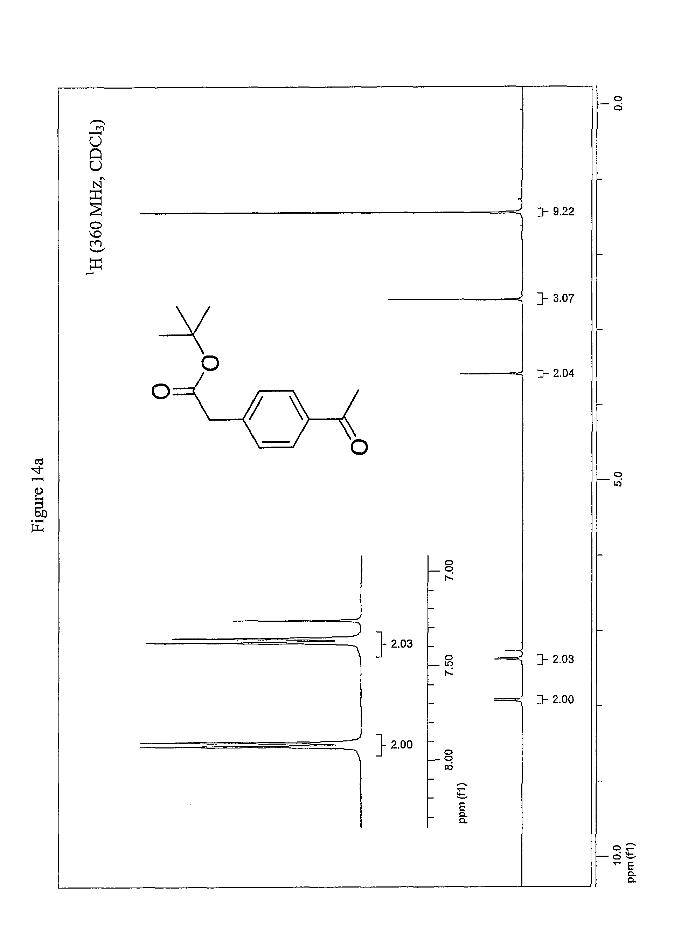

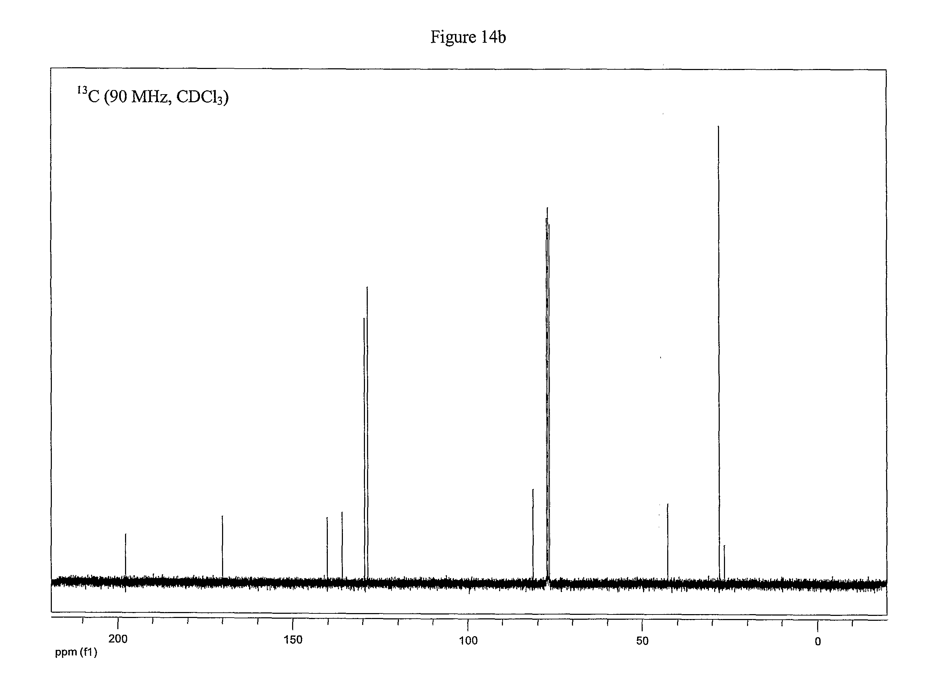

Figure 14 shows the NMR spectra of *tert*-butyl 2-(4-acetylphenyl)acetate (**6**);

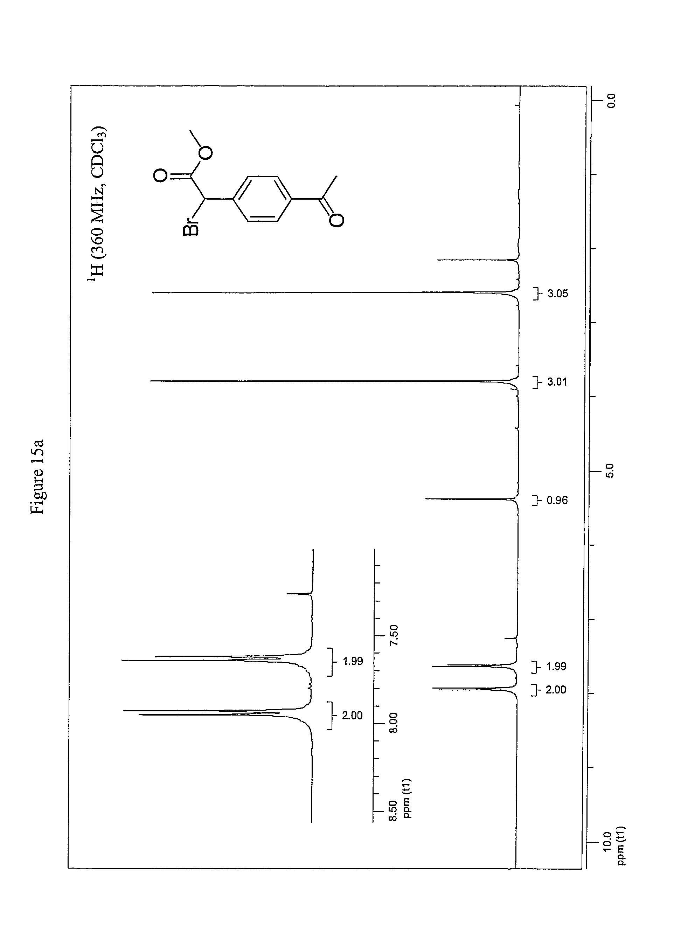

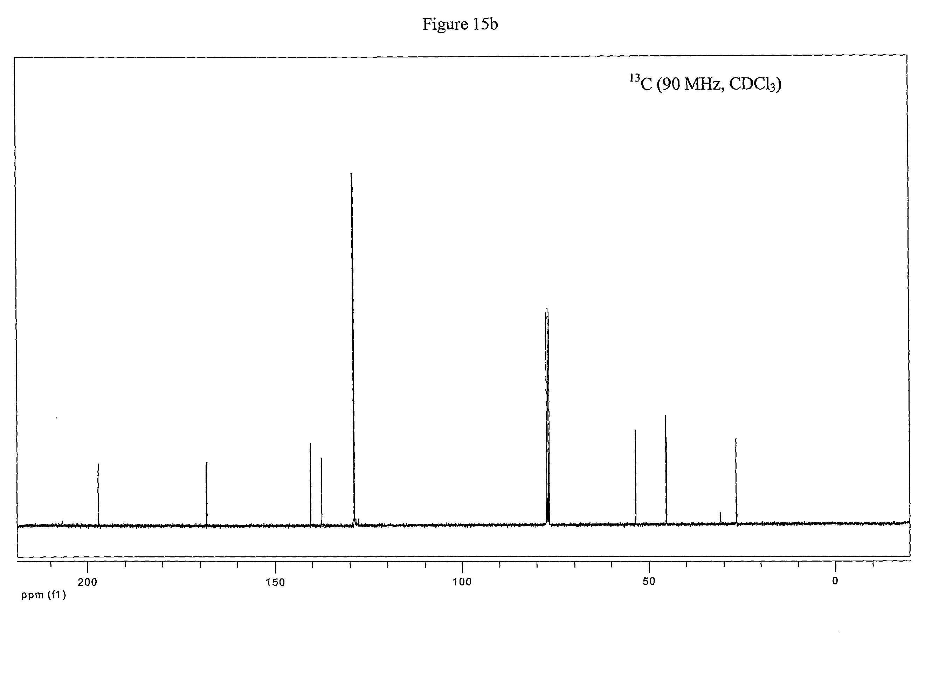

Figure 15 shows the NMR spectra of methyl 2-(4-acetylphenyl)-2-bromoacetate (**7**);

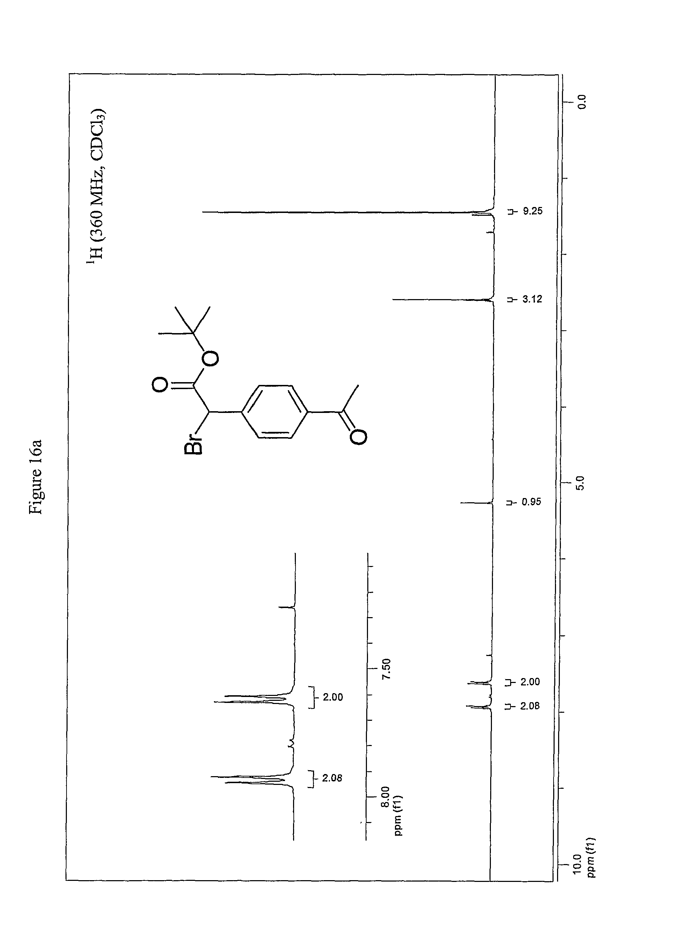

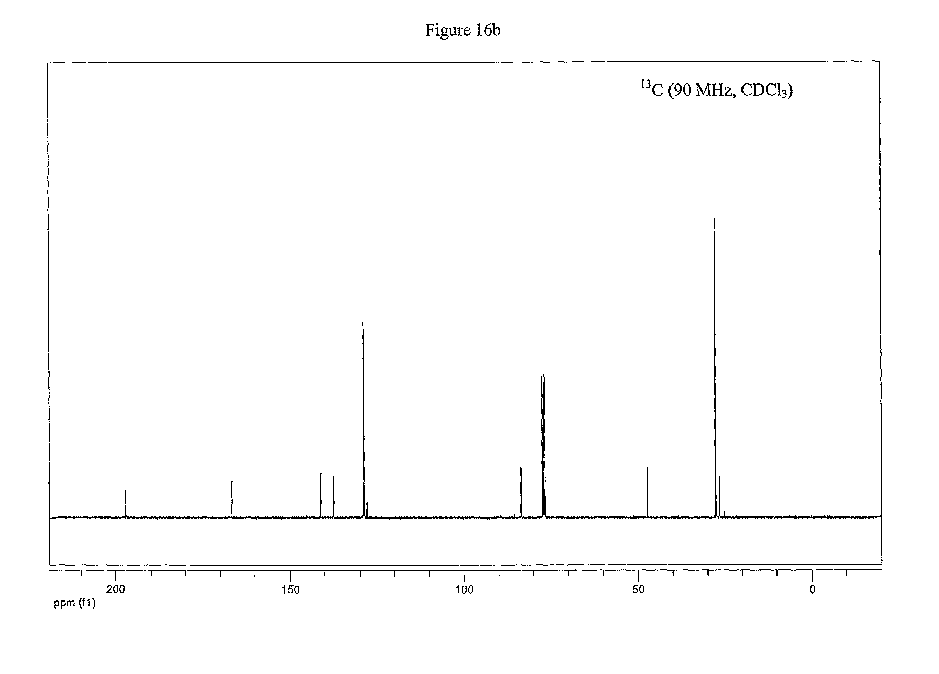

Figure 16 shows the NMR spectra of *tert*-butyl 2-(4-acetylphenyl)-2-bromoacetate (**8**);

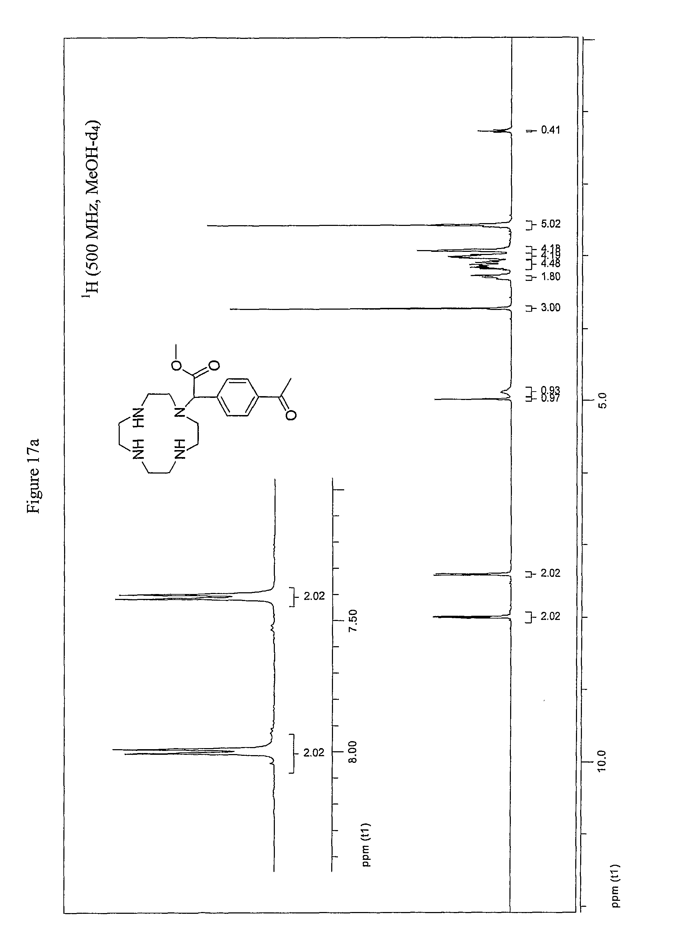

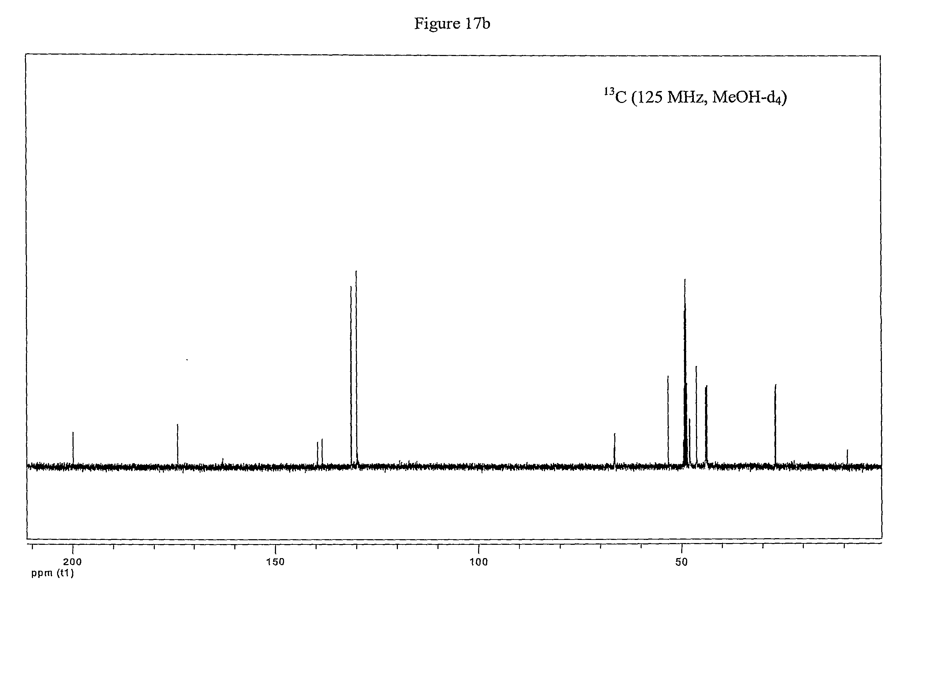

Figure 17 shows the NMR spectra of methyl 2-[1-(1,4,7,10-tetraazacyclododecane)]-(4-acetylphenyl)acetate (**9**);

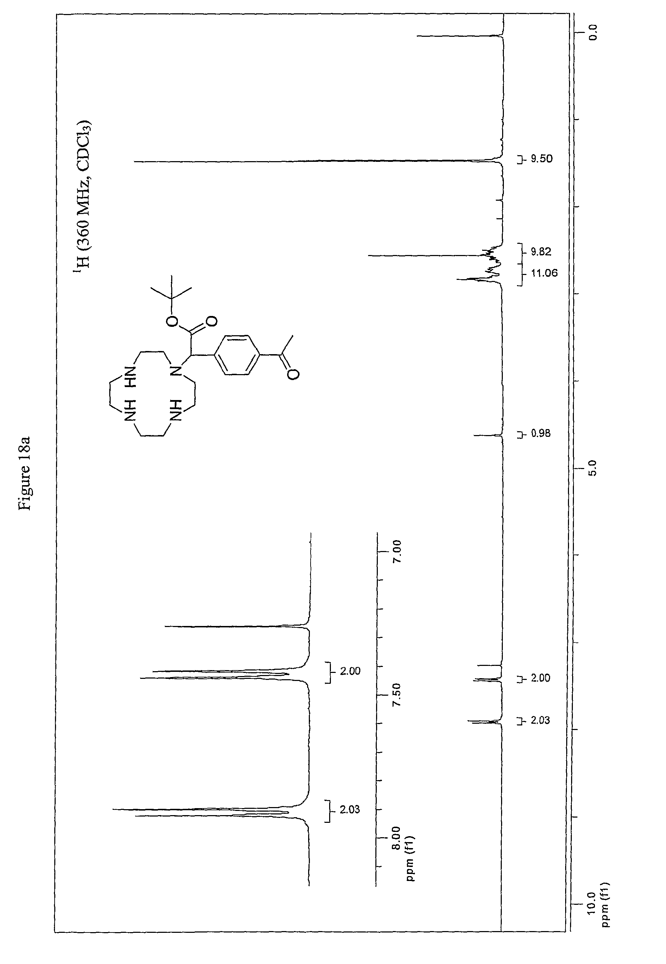

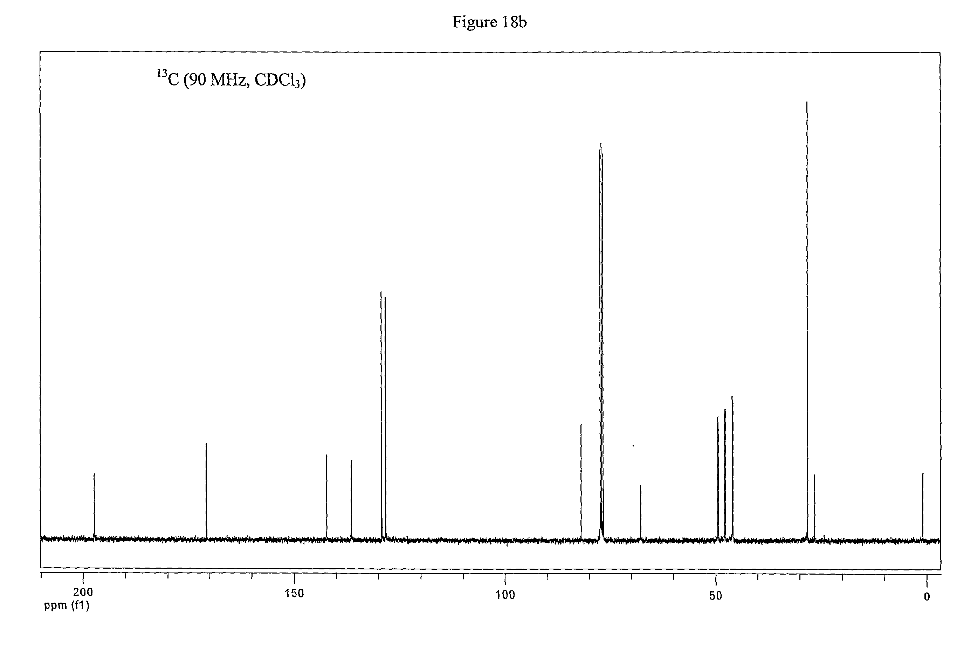

Figure 18 shows the NMR spectra of *tert*-butyl 2-[1-(1,4,7,10-tetraazacyclododecane)] -(4-acetylphenyl)acetate (**10**);

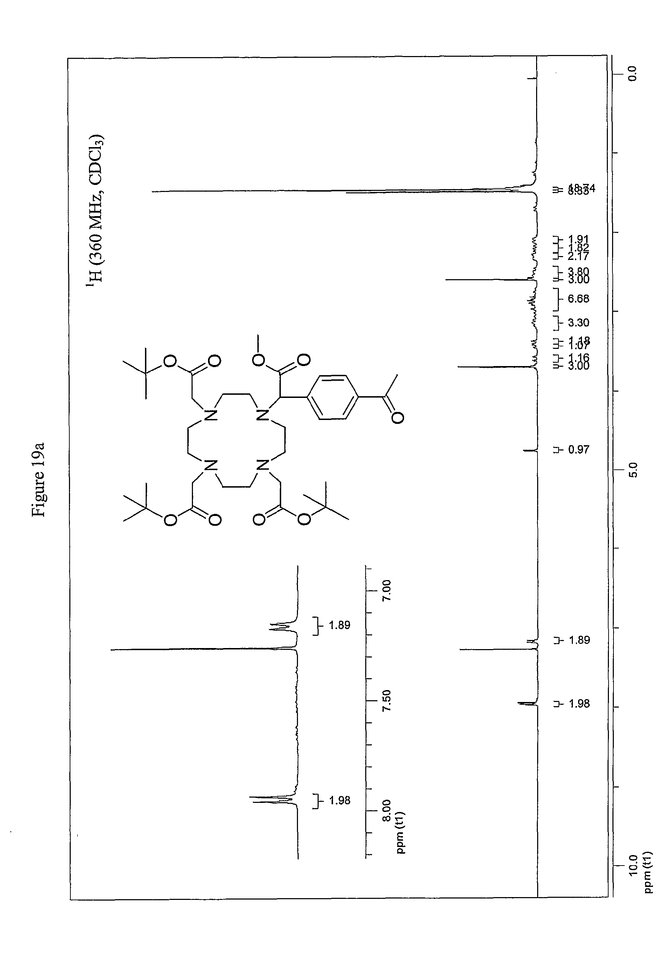

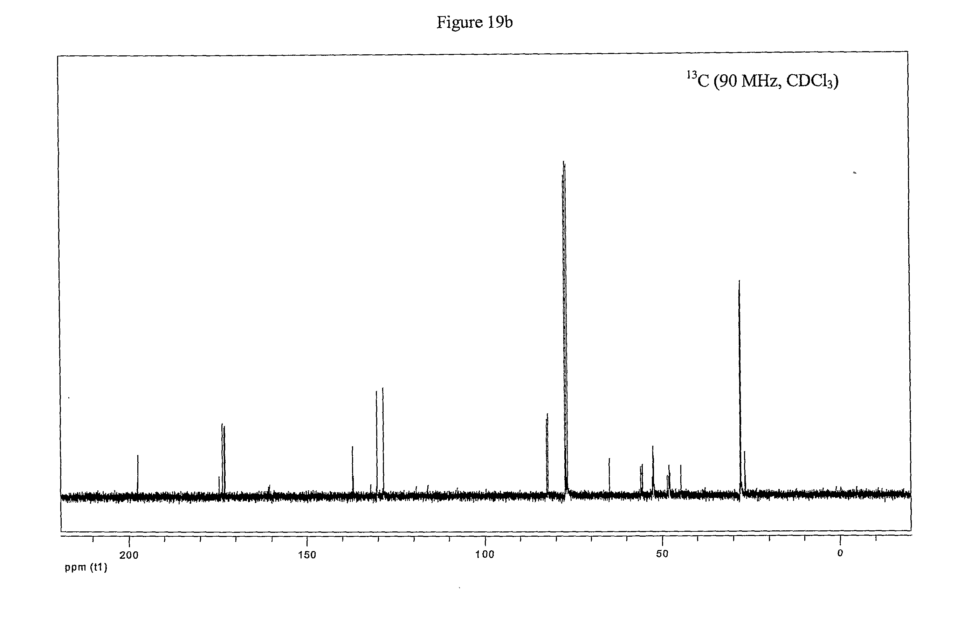

Figure 19 shows the NMR spectra of methyl 2-[1-(1,4,7,10-tetraazacyclododecane)-4,7,10-tris(*tert*-butylacetate)]-(4-acetylphenyl)acetate (**11**);

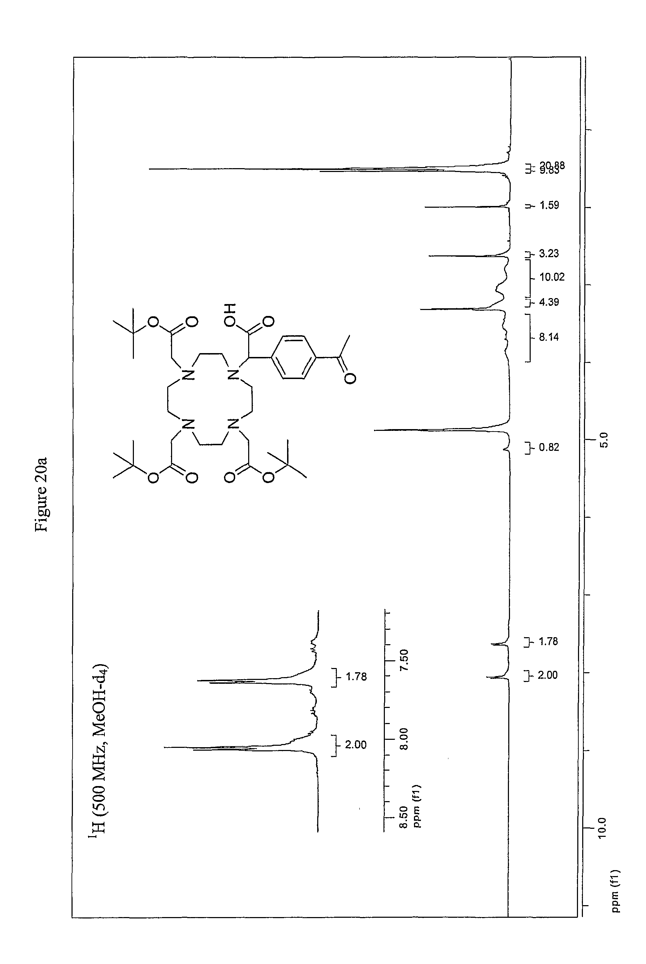

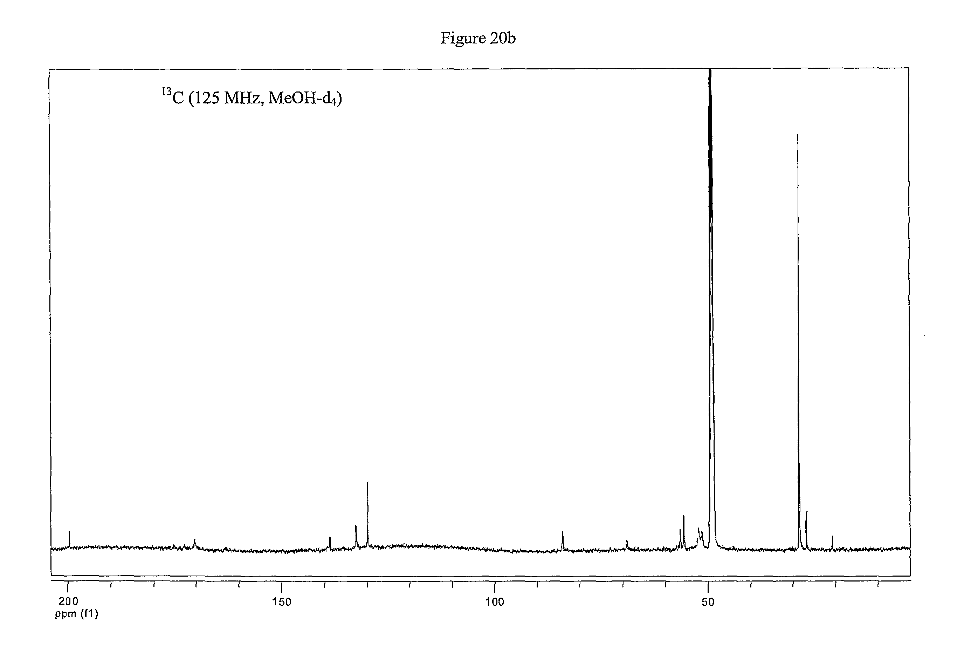

Figure 20 shows the NMR spectra of 2-[1-(1,4,7,10-tetraazacyclododecane)-4,7,10-tris(*tert*-butylacetate)]-(4-acetylphenyl) acetic acid (**2**);

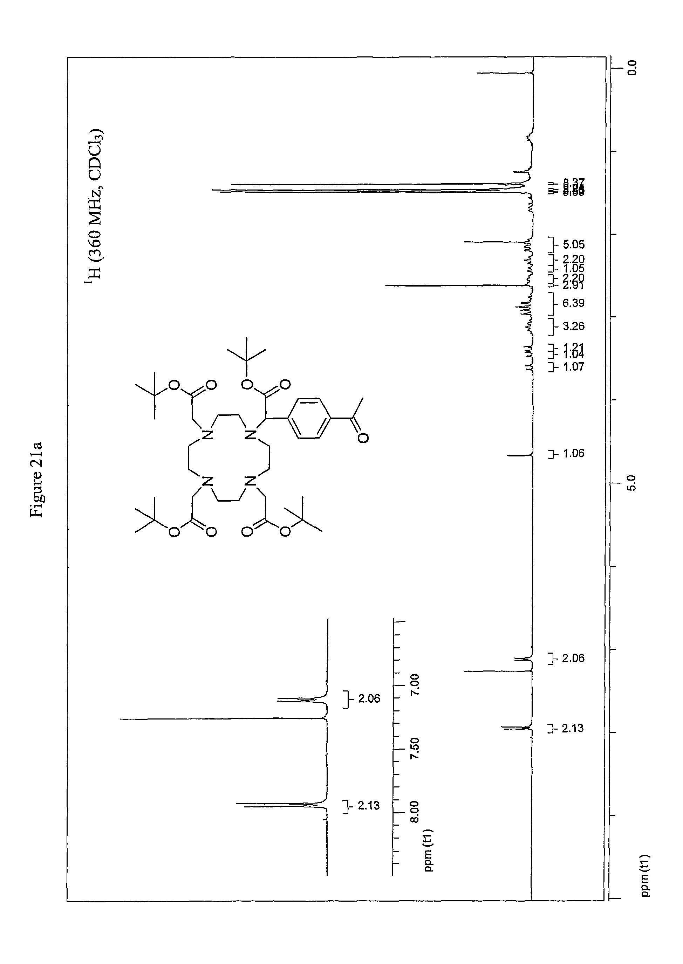

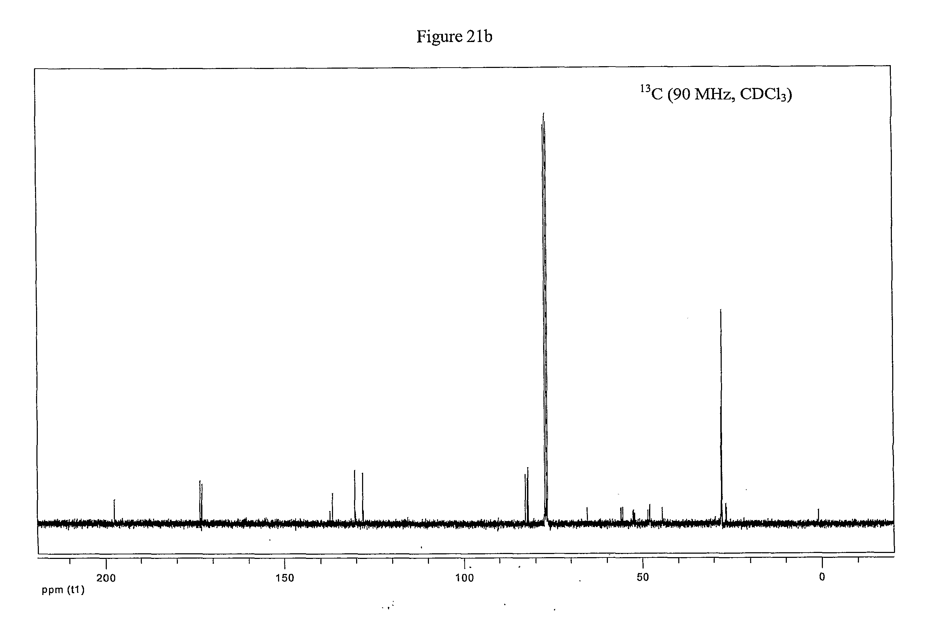

Figure 21 shows the NMR spectra of *tert*-butyl 2-[1-(1,4,7,10-tetraazacyclododecane)-4,7,10-tris(*tert*-butylacetate)]-(4-acetylphenyl)acetate (**1**);

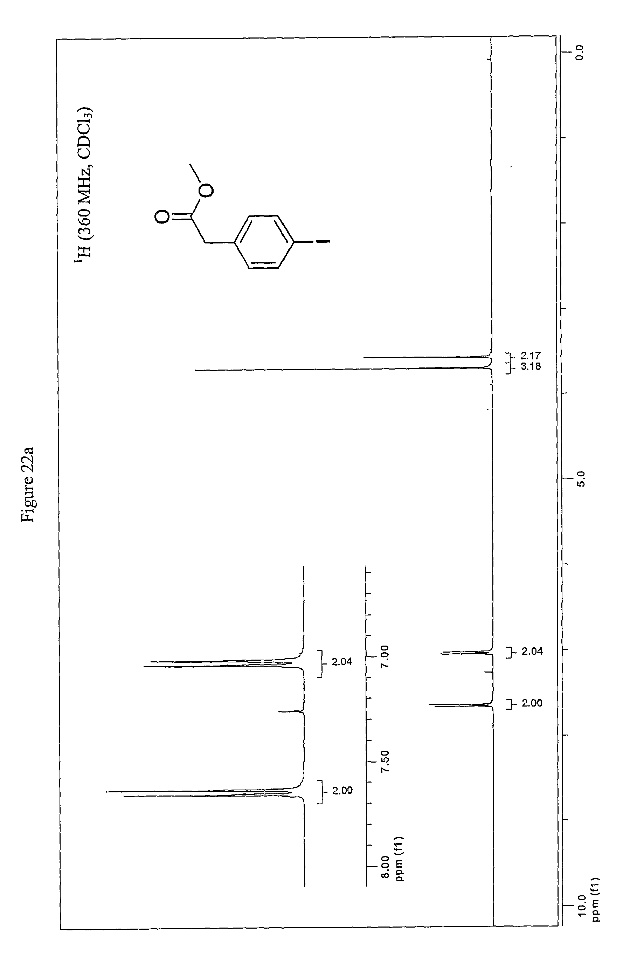

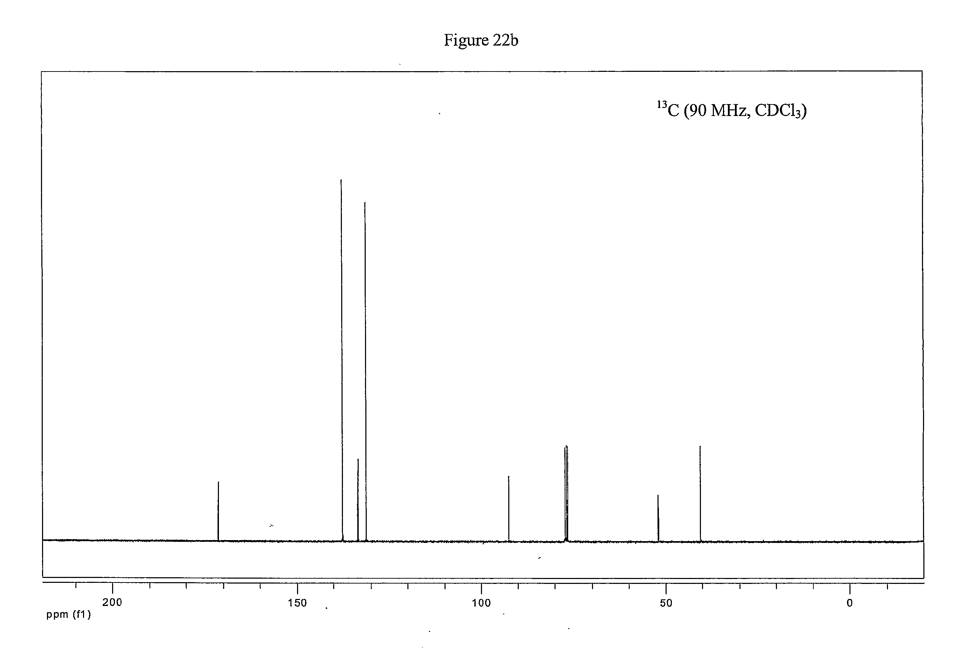

Figure 22 shows the NMR spectra of Methyl (4-iodophenyl)acetate (**13**);

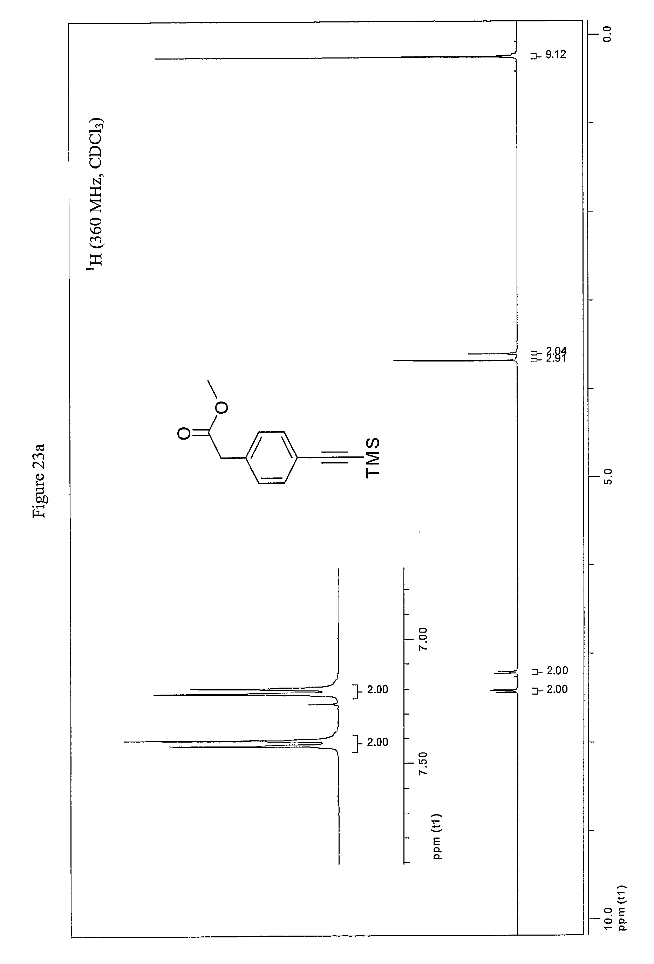

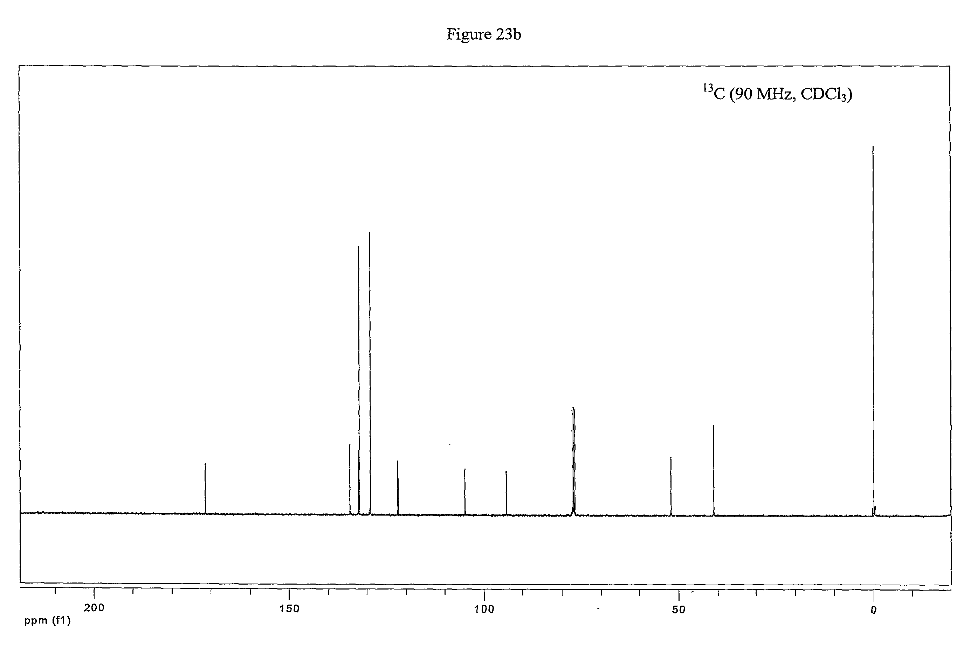

Figure 23 shows the NMR spectra of Methyl 2-(4-(2-(trimethylsilyl)ethynyl)phenyl)acetate (**14**);

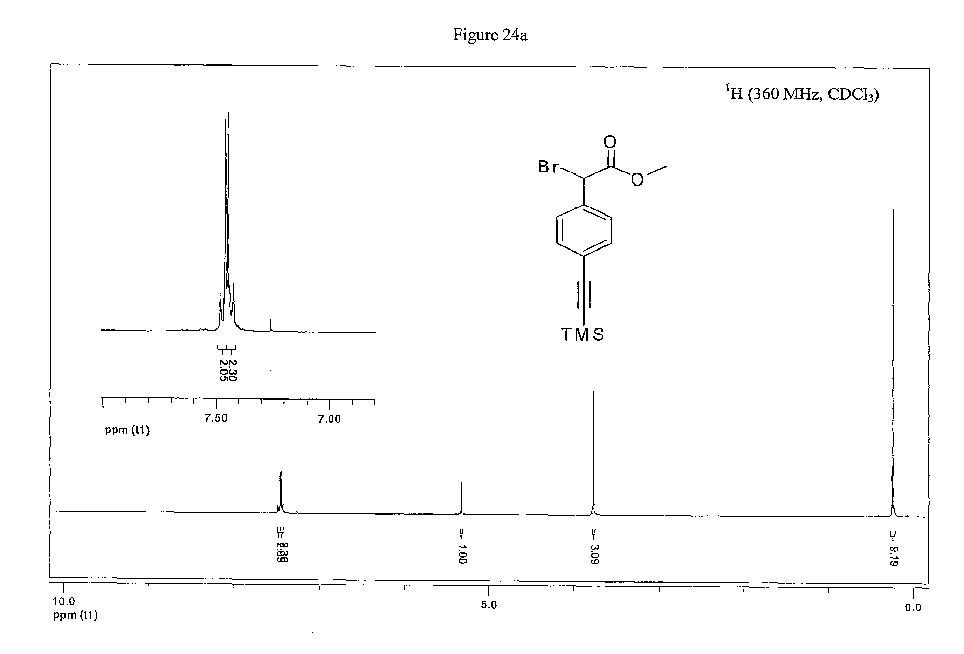

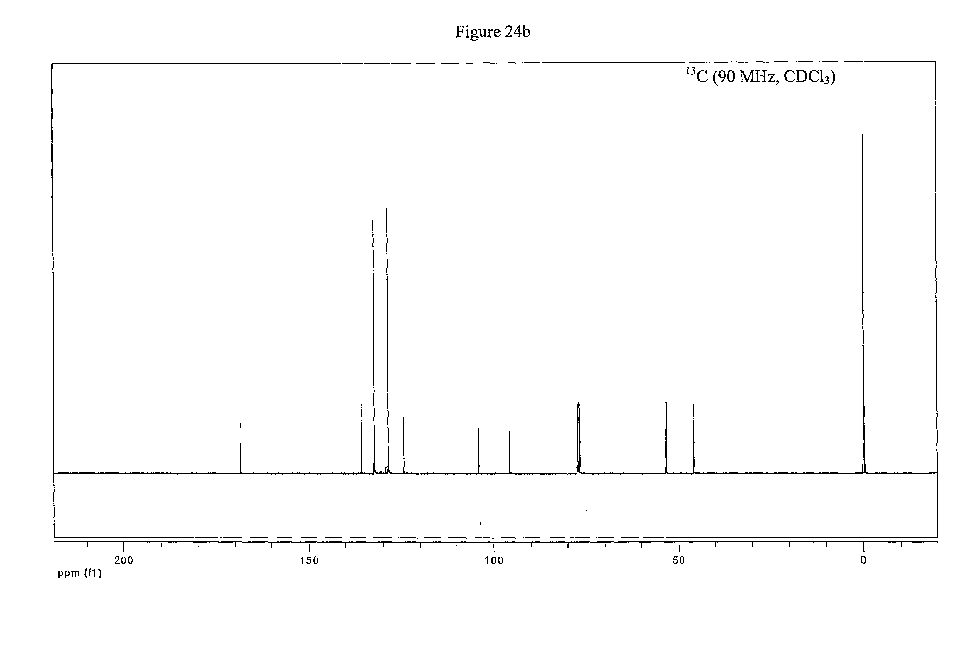

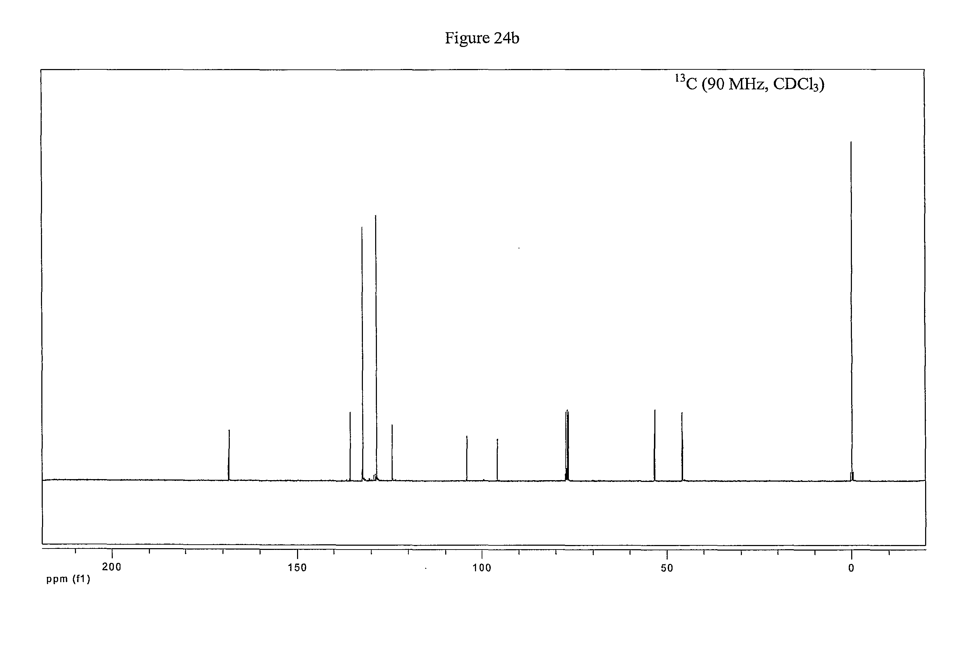

Figure 24 shows the NMR spectra of Methyl 2-bromo-2-(4-(2-(trimethylsilyl)ethynyl)phenyl)acetate (**15**);

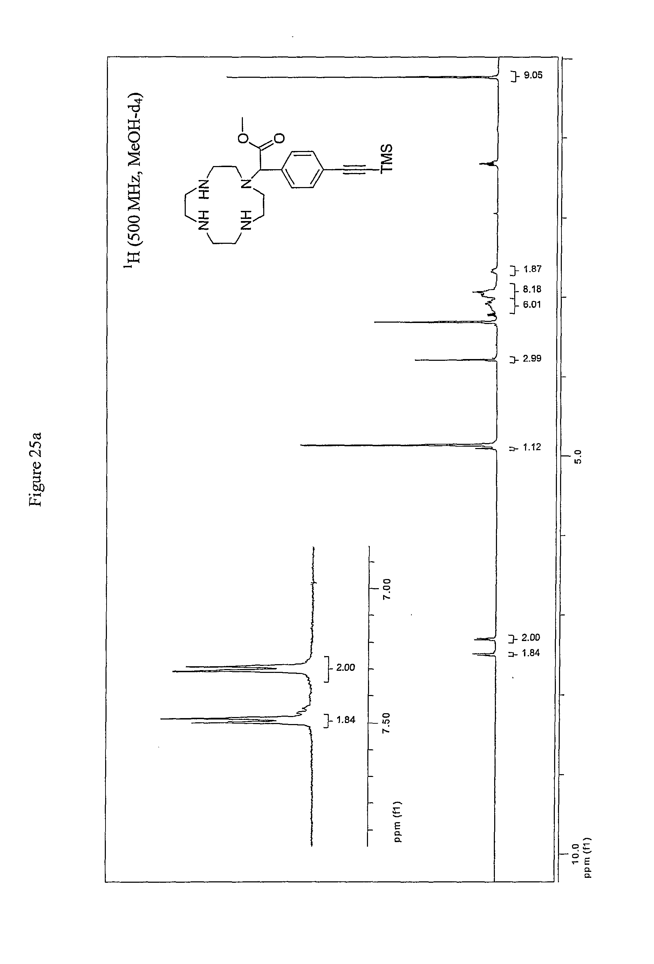

Figure 25 shows the NMR spectra of (*R*/*S*)-methyl 2-[1-(1,4,7,10-tetraazacyclododecane)]-2-(4-ethynyl)phenyl)acetate (**16**);

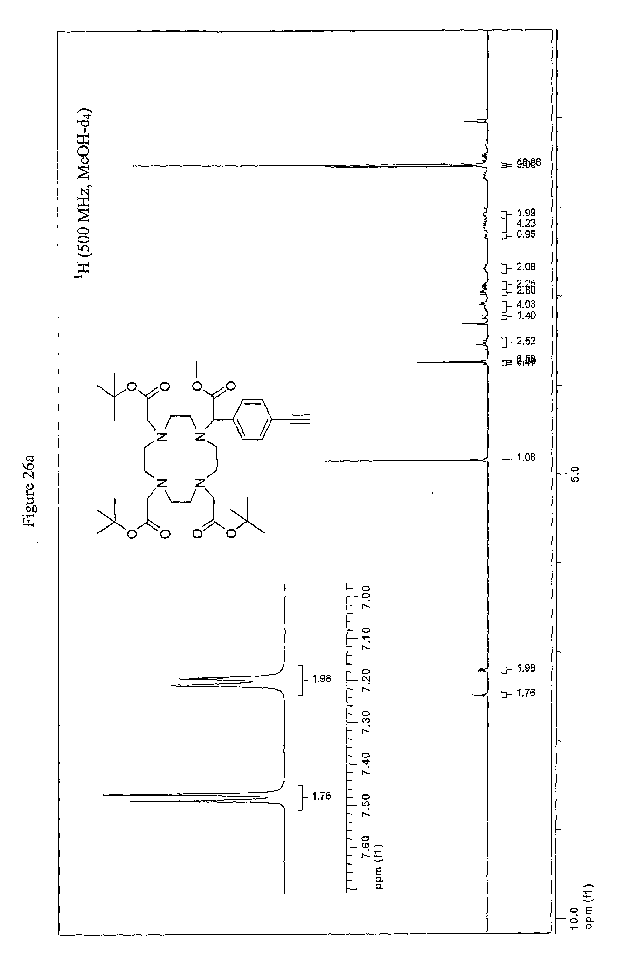

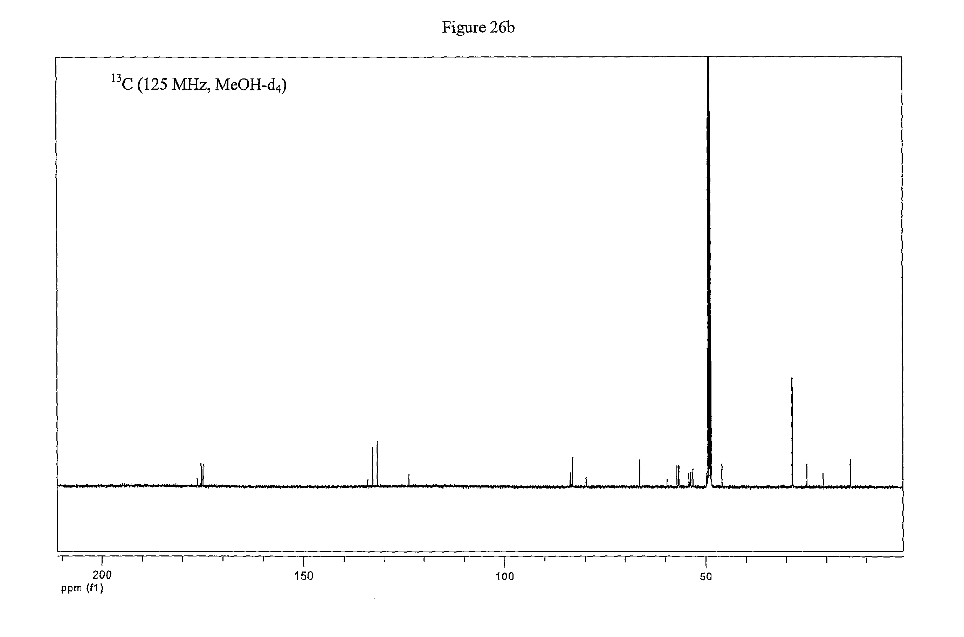

Figure 26 shows the NMR spectra of (*R*/*S*)-methyl 2-[1-(1,4,7,10-tetraazacyclododecane)-4,7,10-tris(*tert*-butylacetate)]-2-(4-ethynyl)phenyl)acetate (**3**); and

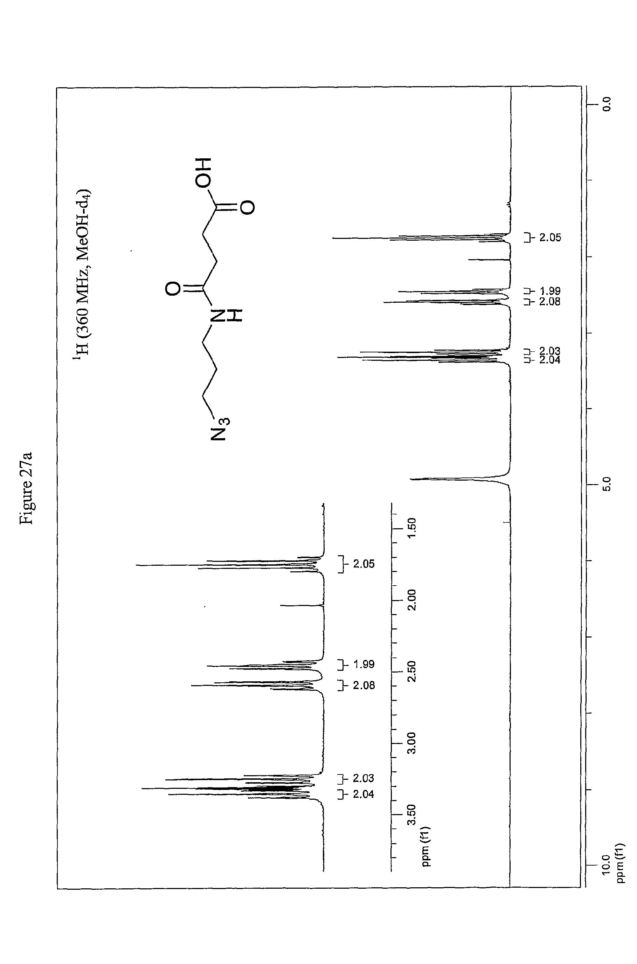

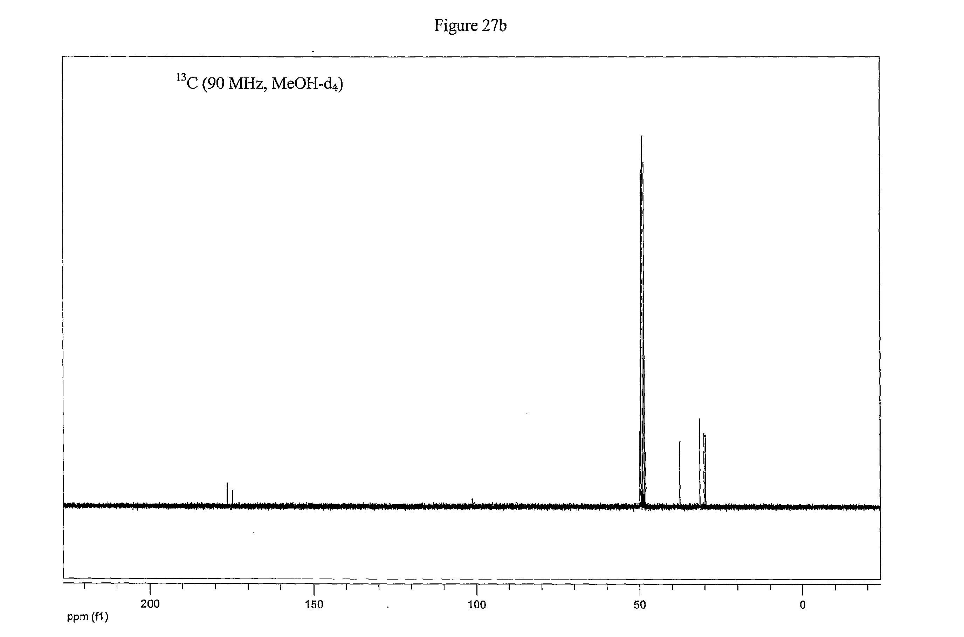

Figure 27 shows the NMR spectra of *N*-(3-azidopropyl)succinamide (**21**).

A convenient synthesis of novel bifunctional poly(amino carboxylate) chelating agents allowing chemoselective attachment to highly functionalized biomolecules is described. Based on the well known chelator 1,4,7,10-tetraazacyclododecane-1,4,7,10-tetracetic acid (DOTA), novel bifunctional chelating agents were synthesized bearing additional functional groups by alkylating 1,4,7,10-tetraazacyclododecane (cyclen) with one equivalent of para-functionalized alkyl 2-bromophenylacetates and three equivalents of commercially available alkyl 2-bromoacetates. The resulting compounds having an additional carbonyl and alkyne functionality respectively allow site specific labelling of unprotected and appropriate functionalized biomolecules in a rapid manner. This was demonstrated by the attachment of our new DOTA-derivatives to the somatostatin analogue Tyr³-octreotate by chemoselective oxime ligation and 1,3-dipolar cycloaddition ("click chemistry"). Initial biodistribution studies in mice demonstrated the suitability of the described DOTA conjugation for in vivo imaging studies with radiometalated peptides.

Thus, our goal was to develop novel DOTA-derivatives enabling the chemoselective attachment in presence of a wide range of functional groups. Thereby, the focus was on structures easily accessible in few synthetic steps from cheap commercial materials to make our method convenient and even a general alternative to the widely used but quite expensive DOTA-tris(*tert*-butyl) ester. Furthermore, the accessibility of the corresponding polyfunctionalized compounds was an important aspect in our consideration. The oxime ligation^{16,17} as well as the Huisgen 1,3-dipolar cycloaddition^{18,19} are well-studied and powerful reactions for chemoselective couplings which fulfill all requirements for our purpose. The oxime ligation denotes the highly selective reaction between an aminooxy component and aldehydes or methylketones²⁰ under formation of an oxime bond, which is known to be reasonably stable both *in vitro* and *in vivo*. The reaction was shown to tolerate every free amino acid side chain except an *N*-terminal cysteine and found widespread use, *e*.*g*. in the synthesis of template assembled synthetic proteins^{21,22}, radioactive labeled peptide conjugates,^{23,24} cyclic peptides²⁵ and protein analogues.^{26,27} The chemoselective Huisgen 1,3-dipolar cycloaddition of an azide and an alkyne for the formation of a triazole was recently rediscovered as a highly useful "click" reaction²⁸⁻³⁰ and has found application in various developments in medicinal chemistry.³¹⁻³⁶ To provide users with different methodologies for the synthesis of DOTA-conjugates we focused on appropriate DOTA-derivatives for both reaction types described above.

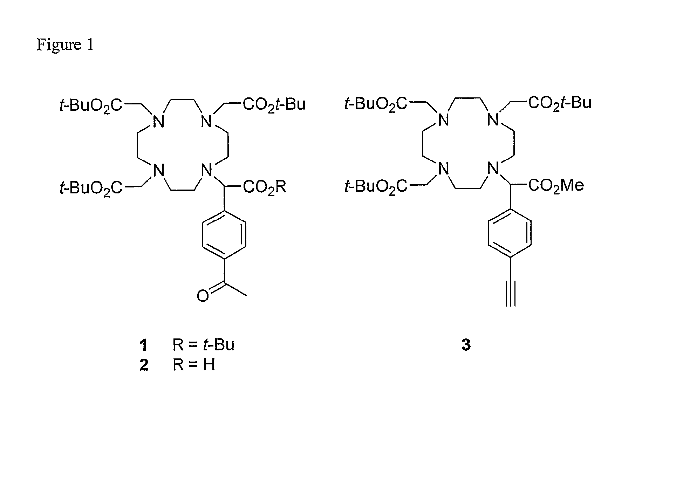

Thus, in this report we present the synthesis of the compounds 2-[1-(1,4,7,10-tetraazacyclododecane)-4,7,10-tris(*tert*-butylacetate)]-(4-acetylphenyl)acetic acid (**1**) and 2-[1-(1,4,7,10-tetraazacyclododecane)-4,7,10-tris(*tert*-butylacetate)]-(4-acetylphenyl)acetic acid *tert-butyl* ester (**2**), respectively, as carbonyl components for oxime ligations with aminooxy-functionalized compounds and 2-[1-(1,4,7,10-tetraazacyclodecane)-4,7,10-tris(*tert*-butylacetate)]-(4-trimethylsilylethinyl)acetic acid methyl ester (**3**) for "click" reactions with azide functionalized compounds (Figure 1). In addition, to attach these derivatives via their methylketone- and alkyne-functionality to biomolecules, compounds **2** and **3** would allow further selective modification due to one differently functionalized carboxyl group. Both classes of derivatives proved to react highly selectively with appropriate N-terminally modified unprotected Tyr³-octreotate, a somatostatin analogue which is a well established targeting molecule for tumor diagnostics and endoradiotherapeutic purposes.³⁷⁻⁴⁰ To demonstrate the applicability of the afore mentioned different chemoselective conjugation approaches for the synthesis of new radioligands without affecting the receptor affinity of the respective biomolecule, one of the Tyr³-octreotate analogs was labeled with ⁶⁸Ga and evaluated in an initial biodistribution study in AR42J tumor bearing nude mice.

### Experimental Procedures

**General.** Tritylchloride polystyrol (TCP) resin (0.94 mmol/g) was purchased from PepChem (Tübingen Germany). Coupling reagents and amino acid derivatives were purchased from Novabiochem, Neosystem, and IRIS Biotech GmbH, Merck Biosciences, Perseptive Biosystems GmbH and Neosystem. Dry solvents were purchased from Fluka. All other reagents and solvents were purchased from Merck, Aldrich and Fluka and were used as received. Standard syringe techniques were applied for transferring dry solvents. Thin-layer chromatography (TLC) was performed on aluminium-backed plates Merck silica gel 60 F₂₅₄. Compounds were visualized by UV absorption at 254 nm or coloration with cerium ammonium molybdate (CAM). Flash chromatography was performed on silica gel 60 (Merck, 230-400 mesh) (ca. 50 g for 1 g of material to be separated) with the indicated eluent. Solvents for chromatography were distilled prior to use. Chromatographic elution solvent systems are reported as volume/volume ratios. RP-HPLC analyses was performed using an Omnicrom YMC column (4.6 mm × 250 mm, 5 µm C₁₈, 1 mL/min) and detection at 254 nm for non-peptidic compounds and 220 nm for peptidic compounds. The eluent was a linear gradient from water (0.1% TFA) to acetonitrile (0.1% TFA) over 30 minutes. The retention time (*R*ₜ) of the analytical RP-HPLC is given in minutes with the gradient in percentage of acetonitrile. NMR: Bruker AC-250, AV-360, AV-500 and DMX500. ¹H and ¹³C NMR spectra were recorded at ambient temperature. Spectra were calibrated to their respective solvent signals (CDCl₃: ¹H 7.26 ppm, ¹³C 77.0 ppm; MeOH-d₄: ¹H 3.31 ppm, ¹³C 49.05 ppm;). Chemical shifts (δ) are reported in parts per million (ppm) and coupling constants (*J* values) are given in hertz (Hz). The following abbreviations were used to explain the multiplicities: s, single; d, doublet; t, triplet; q, quartet; dd, doublet of doublets; dt, doublet of triplets; m, multiplet; b, broad. MS: Finnigan MAT 8200 (EI), Finnigan LCQ (ESI). Peptide sequence analysis was performed on a Bruker Ultraflex TOF/TOF.

**Methyl 2-(4-acetylphenyl)acetate** (5). To a suspension of methyl 2-bromoacetate (3.55 mL, 37.5 mmol, 1.0 equiv), Pd(OAc)₂ (252 mg, 1.13 mmol, 3 mol%), P(*o*-tolyl)₃ (1.02 g, 3.35 mmol, 9 mol%) and K₂CO₃ (26.0 g, 0.19 mmol, 5.0 equiv) in THF (120 mL) under argon was added a solution of 4-acetylphenylboronic acid (7.38 g, 45.0 mmol, 1.2 equiv) in THF/H₂O (120 mL/1.7 mL) drop wise over 1 h. After stirring for 18 h at room temperature, the mixture was filtered and the solvent removed under reduced pressure. The residue was taken up in EtOAc (250 mL) and subsequently washed with saturated aqueous NH₄Cl (150 mL), saturated aqueous NaHCO₃ (150 mL) and brine. After drying over MgSO₄, the organic layer was filtered through a short column of silica gel and concentrated. Flash chromatography on silica gel (EtOAc/hexane 1:8) yielded **5** (4.73 g, 66%) as a pale yellow solid. *R*_{f}=0.13 (EtOAc/hexane, 1:4); mp 44-44°C; ¹H NMR (360 MHz, CDCl₃) δ 7.91 (d, *J*=8.3 Hz, 2H), 7.36 (d, *J*=8.3 Hz, 2H), 3.69 (s, 3H), 3.68 (s, 2H), 2.57 (s, 3H); ¹³C NMR (90 MHz, CDCl₃) δ 197.5, 171.1, 139.2, 136.0, 129.4 (2C), 128.5 (2C), 52.1, 40.9, 26.5; HRMS (EI) calcd for C₁₁H₁₂O₃ 192.07864; found 192.07863.

**Methyl 2-(4-acetylphenyl)-2-bromoacetate (7).** To a solution of **5** (3.00 g, 15.6 mmol, 1.0 equiv) in dry CCl₄ (300 mL) was added *N*-bromosuccinimide (3.36 g, 18.9 mmol, 1.2 equiv) and Br₂ (2 drops). The mixture was heated to reflux, illuminate with a 500 W halogen lamp for 10 min and stirred for further 50 min under reflux. After cooling to room temperature, the reaction solution was filtered, the solvent concentrated and the crude product purified by flash chromatography on silica gel (EtOAc/hexane 1:4) to give **7** (3.75 g, 89%) as a pale yellow oil. *R*_{f}=0.18 (EtOAc/hexane, 1:4); ¹H NMR (360 MHz, CDCl₃) δ 7.94 (d, *J*=8.4 Hz, 2H), 7.63 (d, *J*=8.4 Hz, 2H), 5.38 (s, 1H), 3.79 (s, 3H), 2.59 (s, 3H); ¹³C NMR (90 MHz, CDCl₃) δ 197.1, 168.2, 140.4, 137.5, 128.9, 128.6, 53.5, 45.3, 26.6; HRMS (EI) calcd for C₁₁H₁₁⁸¹BrO₃ 271.98712; found 271.98716, HRMS (EI) calcd for C₁₁H₁₁⁷⁹BrO₃ 269.98917; found 269.98863.

**(*R*/*S*)-Methyl 2-[1-(1,4,7,10-tetraazacyclododecane)]-2-(4-acetylphenyl)acetate (9).** To a suspension of 1,4,7,10-tetraazacyclododecane (762 mg, 4.43 mmol, 1.2 equiv) and K₂CO₃ (1.27 g, 9.22 mmol, 2.5 equiv) in DMF (100 mL) at room temperature was added a solution of **7** (1.00 g, 3.69 mmol, 1.0 equiv) in DMF (60 mL) drop wise over 10 h. The mixture was filtrated and concentrated under reduced pressure. Flash chromatography on silica gel (gradient MeOH/CHCl₃ 1:7→7:1, 1% NEt₃) yielded **9** (829 mg, 62%) as a pale yellow solid. *R*_{f}=0.10 (MeOH/CHCl₃, 3:1, 1% NEt₃); mp 32-35°C; ¹H NMR (500 MHz, MeOH-d₄) δ 8.04 (d, *J*=8.2 Hz, 2H), 7.45 (d, *J*=8.2 Hz, 2H), 5.02 (s, 1H), 3.77 (s, 3H), 3.37-3.30 (m, 2H), 3.24-3.10 (m, 4H), 3.09-3.01 (m, 4H), 3.00-2.94 (m, 4H), 2.63 (m, 5H); ¹³C NMR (125 MHz, MeOH-d₄) δ 199.8, 174.1, 139.6, 138.4, 131.2 (2 C), 129.8 (2 C), 66.5, 53.2, 48.0 (2 C), 46.3 (2 C), 43.8 (2 C), 43.8 (2 C), 26.8; MS (ESI) calcd for C₁₉H₃₀N₄O₃ 362.2; found 363.2 [M+H]⁺.

**(*R*/*S*)-Methyl 2-[1-(1,4,7,10-tetraazacyclododecane)-4,7,10-tris(*tert*-butylacetate)]-2-(4-acetyl-phenyl) acetate (11).** To a suspension of **9** (723 mg, 1.99 mmol, 1.0 equiv) and K₂CO₃ (1.24 g, 8.98 mmol, 4.5 equiv) in DMF (50 mL) at room temperature was added a solution of tert-butyl 2-bromoacetate (0.97 mL, 6.58 mmol, 3.3 equiv) in DMF (50 mL) over 30 min. After stirring for 4 h, the mixture was filtrated and concentrated under reduced pressure. Flash chromatography on silica gel (MeOH/CHCl₃ 1:10→7:1, 1% NEt₃) yielded **11** (83 mg, 83%) as a pale yellow solid. 99% purity; RP-HPLC (10→80%) *R*ₜ=17.4; *R*_{f}=0.83 (MeOH/CHCl₃, 3:1, 1% NEt₃); mp 73-75°C; ¹H NMR (360 MHz, CDCl₃) δ 7.95 (d, *J*=8.3 Hz, 2H), 7.16 (d, *J*=8.1 Hz, 2H), 4.75 (s, 1H), 3.69 (s, 3H), 3.59 (d, *J*=17.4 Hz, 1H), 3.42 (d, *J*=17.5 Hz, 1H), 3.39 (d, *J*=17.4 Hz, 1H), 3.24-3.05 (m, 3H), 3.02-2.68 (m, 6H), 2.59 (s, 3H), 2.57-2.42 (m, 4H), 2.35-2.25 (m, 2H), 2.25-2.15 (m, 2H), 2.14-2.05 (m, 2H), 1.48 (s, 9H), 1.45 (s, 18H); ¹³C NMR (90 MHz, CDCl₃) δ 197.4, 174.5, 173.6, 173.1, 172.9, 136.9, 136.7, 130.2 (2C), 128.3 (2C), 82.5, 82.2, 82.1, 77.2, 64.8, 55.9, 55.7, 55.5, 52.7, 52.4, 52.3, 48.5, 48.0, 47.9, 47.8, 44.6, 27.9 (3C), 27.8 (3C), 27.7 (3C), 26.6; HRMS (EI) calcd for C₂₂H₃₆N₄O₃ 404.27875; found 404.27951.

**(*R*/*S*)-2-[1-(1,4,7,10-Tetraazacyclododecane)-4,7,10-tris(*tert*-butylacetate)]-2-(4-acetylphenyl)-acetic acid * ½AcOH (2).** To a solution of **11** (26.0 mg, 31.7 µmol, 1.0 equiv) in THF (5 mL) at room temperature was added a solution of LiOH (1.75 mg, 72.9 µmol, 2.3 equiv) in water (150 µL) and the mixture was stirred for 18 h. After concentration under reduced pressure, the crude product was purified by preparative RP-HPLC (20→60%, 30 min) and lyophilized out of acetic acid to yielded **2** (9.0 mg, 38%, 66% related to recovered **11**) as a colorless solid. 94% purity; RP-HPLC (10→60%) *R*ₜ=25.7; mp 68-72°C; ¹H NMR (500 MHz, MeOH-d₄) δ 8.06 (d, *J*=8.2 Hz, 2H), 7.63 (d, *J*=8.1 Hz, 2H), 5.12 (s, 1H), 4.04-3.34 (m, 8H), 3.34-3.18 (m, 4H), 3.15-2.66 (m, 10H), 2.63 (s, 3H), 1.99 (s, ½×3H (AcOH)), 1.53 (s, 9H), 1.52 (s, 18H); ¹³C NMR (125 MHz, MeOH-d₄) δ 199.7, 175.2, 172.7, 170.4 (br, 3C), 138.8 (2C), 132.7 (2C), 129.8 (2C), 84.1 (br, 3C), 69.9, 56.5, 52.9-51.0 (br, 8C), 20.5 (9C), 26.8, 20.8; MS (ESI) calcd for C₃₆H₅₈N₄O₉ 690.4; found 691.4 [M+H]⁺, 713.4 [M+Na]⁺, 729.3 [M+K]⁺.

**Methyl (4-iodophenyl)acetate (13).** To a solution of (4-iodophenyl)acetic acid (**12**) (15.0 g, 57.0 mmol) in dry methanol (50 mL) was added SOCl₂ (20.7 mL, 285 mmol, 5 equiv) drop wise at 0°C. After stirring for 1 h at room temperature, the solvent was removed under reduced pressure and the residue dissolved in Et₂O (400 mL). The organic phase was subsequently washed with saturated aqueous NaHCO₃ (400 mL), saturated aqueous NH₄Cl (400 mL) and brine (400 mL) and dried over MgSO₄. Concentration under reduced pressure gave **13** (14.7 g, 93%). *R*_{f}=0.57 (EtOAc/hexane, 1:4); ¹H NMR (360 MHz, CDCl₃) δ 7.65 (d, *J*=8.3 Hz, 2H), 7.03 (d, *J*=8.3 Hz, 2H), 3.69 (s, 3H), 3.56 (s, 3H); ¹³C NMR (90 MHz, CDCl₃) δ 171.3, 137.6, 133.5, 131.2, 92.5, 52.0, 40.5.

**1-Azido-3-Aminopropane (20).** The literature procedure (*56*) was slightly modified: A solution of 1-bromo-3-aminopropane hydrobromide (5.47 g, 25.0 mmol, 1.0 equiv) and sodium azide (3.25 g, 50.0 mmol, 2.0 equiv) in 20 mL water was heated at 80 °C for 24 h. The reaction mixture was cooled in an ice bath followed by addition of diethyl ether (30 mL). The pH was adjusted to 14 by addition of KOH pellets keeping the temperature below 10 °C. After separation of the organic phase the aqueous was further extracted with diethyl ether. The combined organic layers were dried over MgSO₄ and carefully concentrated in vacuo. The remaining oil contained **20** 21.0 mmol (84%) and about equimolar amounts of diethyl ether (based on integration of proton NMR) and was used without further purification. Spectroscopical data was identical to literature **20**.

**3-(3-Azidopropylcarbamoyl)propanoic acid (21).** To a solution of **20** (501 mg, 5.00 mmol, 1.1 eq) and NEt₃ (693 µL, 5.00 mmol, 1.1 eq) in 10 mL acetone was added a solution of succinic anhydride (460 mg, 4.55 mmol, 1.0 eq) in 5 mL acetone over a period of 15 min at room temperature. The reaction mixture was stirred for further 15 hours at room temperature. After concentration in vacuo the residue was partitioned between diluted aqueous HCl (20 mL) and ethyl acetate (20mL), separated and the aqueous layer was further extracted with ethyl acetate (2 x 10 mL). After concentration of the combined organic layers **21** (653 mg, 3.27 mmol, 71 %) was obtained as a white solid. ¹H NMR (360 MHz, MeOH-d₄) δ 3.34 (t, *J*=6.8 Hz, 2H), 3.24 (t, *J*=6.8 Hz, 2H), 2.59 (dt, *J*=6.5 Hz, 1.1 Hz, 2H), 2.44 (dt, *J*=6.7 Hz, 1.3 Hz, 2H), 1.74 (q, *J*=6.8 Hz, 2H); ¹³C NMR (90 MHz, MeOH-d₄) δ 176.2, 174.7, 50.0, 37.7, 31.5, 30.2, 29.7; MS (ESI) calcd for C₁₁H₁₂O₃ 200.1; found 201.1 (M+H⁺).

### Peptide Synthesis

Peptide synthesis was carried out using TCP-resin following standard Fmoc-strategy *(57-60).*

### Attachment of N-Fmoc-amino acids to TCP resin. General procedure I.

After swelling with dry DCM (10 mL) for 20 min, the TCP resin (2.00 g, theoretical 0.96 mmol/g, 1.92 mmol) was treated with a solution of Fmoc-protected amino acids (1.2 equiv, 2.3 mmol) in dry DCM (19 mL) and DIPEA (980 µL, 3 equiv, 5.8 mmol) at room temperature for 2 h. MeOH (2 mL) and DIPEA (0.4 mL) were added to cap the free sites, and the reaction mixture was shaken for 15 min. The resin was washed with NMP (3×10 mL), DCM (5×10 mL) and MeOH (3×10 mL) and dried under vacuo to give resin bound *N*-Fmoc-amino acids.

### Fmoc deprotection. General procedure II.

The Fmoc-protected resin was treated with 10 mL of a solution of 20% piperidine in NMP (3×10 min) and washed with NMP (5×10 mL).

### Coupling with TBTU/HOBt. General procedure III.

Fmoc-amino acid (2.5 equiv), TBTU (2.5 equiv), HOBt (2.5 equiv) and DIPEA (7 equiv) were dissolved in NMP to give a 0.2 mmol/L solution which was added to the resin. The reaction mixture was shaken at room temperature for 90 min and washed with NMP (5×10 mL).

### Cleavage and deprotection with trifluoroacetic acid. General procedure IV.

The resin was washed with DCM (3×10 mL) and treated with a mixture of TFA, H₂O and triisopropylsilane (90:5:5, v/v/v; 20 mL) for 3×10 min. After removal of the resin by filtration, the filtrates were combined and stirred for another 90 min. The solvent was concentrated under reduced pressure to precipitate the peptide with Et₂O.

### Disulfide cyclization of the linear peptide. General procedure V.

The linear peptide was dissolved in high dilution (c=1 mM) in water and the pH was adjusted to 7-8 by addition of concentrated aqueous NaHCO₃. Then, 30% aqueous H₂O₂ (3 equiv) was added drop wise under vigorous stirring. The reaction was stopped after 30 min and the solvent removed by lyophilization.

**Synthesis of c*yclo*[2,7]-AoxAc-D-Phe-Cys-Tyr-D-Trp-Lys-Thr-Cys-Thr-OH (18) and c*yclo*[2,7]-3-(3-azidopropylcarbamoyl)propanoyl-D-Phe-Cys-Tyr-D-Trp-Lys-Thr-Cys-Thr-OH (23).** The linear peptide sequence H-D-Phe-Cys-Tyr-D-Trp-Lys-Thr-Cys-Thr-OH was synthesized on solid phase in a similar manner to the general procedures I-III. A sample was cleaved from the resin following procedure IV and the peptide sequence was confirmed by MALDI-TOF peptide sequence analysis. MS (MALDI) calcd for C₄₉H₆₆N₁₀O₁₂S₂ 1050.43; found 1051.40 [M+H]⁺. For the further procedure the resin was parted into two portions:
1) C*yclo[2,7]-AoxAc-D-Phe-Cys-Tyr-D-Trp-Lys-Thr-Cys-Thr-OH (**18**)*: (Boc-aminoxy)acetic acid was coupled to one portion of above resin bound peptide in a similar manner to the general procedures I-III and the linear peptide was cleaved from resin following procedure IV. The disulfide cyclization was accomplished according to general procedure V. Great care must be taken to the quality of all solvents used in steps IV and V (HPLC quality). A small batch of the crude product was purified to obtain the aminooxy-functionalized Tyr³-octreotate **18** as a colorless solid after purification by semipreparative RP-HPLC (20→50%, 30 min). 97% purity; RP-HPLC (10→60%) *R*ₜ=17.3; MS (ESI) calcd for C₅₁H₆₇N₁₁O₁₄S₂ 1121.43; found 1122.5 [M+H]⁺, 562.1 [(2M+2H)/2]²⁺.
*2) Cyclo(2,7)-3-(3-Azidopropylcarbamoyl)propanoyl-D-Phe-Cys-Tyr-D-Trp-Lys-Thr-Cys-Thr-OH (**23**).* **21** was coupled to one portion of above resin bound peptide in a similar manner to the general procedures I-III and the linear peptide was cleaved from resin following procedure IV. The disulfide cyclization was accomplished according to general procedure V. The disulfide cyclization was accomplished according to general procedure V. The peptide was obtained as a colorless solid after purification by semipreparative RP-HPLC (20→60%, 30 min). 96% purity; RP-HPLC (10→50%) *R*ₜ=25.0; MS (ESI) calcd for C₅₆H₇₄N₁₄O₁₄S₂ 1230.50; found 1231.6 [M+H]⁺, 1253.6 [M+Na]⁺, 1269.3 [M+K]⁺.

**Synthesis of 3-(3-Azidopropylcarbamoyl)propanoyl-Tyr-Glu-Trp-Lys (25):** The linear peptide was synthesized on solid phase in a similar manner to the general procedures I-III and cleaved from resin following procedure IV. The peptide was obtained as a colorless solid after purification by semipreparative RP-HPLC (20→50%, 30 min). 97% purity; RP-HPLC (10→60%, 30 min) *R*ₜ=16.8; MS (ESI) calcd for C₃₈H₅₀N₁₀O₁₀ 806.37; found 807.6 [M+H]⁺, 829.5 [M+Na]⁺.

**Synthesis of DOTA-Tyr-Glu-Trp-Lys derivative 26.** To a solution of **3** (3.1 mg, 4.1 µmol, 1.0 equiv) in THF (0.2 mL) at room temperature was added a solution of LiOH (0.3 mg, 14 µmol, 3.4 equiv) in water (30 µL) and the mixture was stirred for 18 h. Then, water (0.2 mL), peptide **22** (3.8 mg, 4.1 µmol, 1.0 equiv), 0.1M aqueous CuSO₄ (49 µL, 4.9 µmol, 1.2 equiv) and copper powder (10 mg) were added subsequently and the mixture stirred for 18 h. After this time, the copper powder was filtered off and dissolved copper salts were precipitated by addition of Na₂S (Na₂S * 9H₂O; 12 mg, 49 µmol, 12.0 equiv). The mixture was filtrated and the solvent removed under reduced pressure. The *tert-butyl* esters were cleaved by treating with a TFA/TIPS/H₂O mixture (95:5:5, v/v/v; 1 mL) for 2 h. Thereafter, the solution was concentrated under reduced pressure and the crude product was directly purified by semipreparative RP-HPLC (20→50%, 30 min) to yield **23** (3.22 mg, 51%) as a colorless powder after lyophilization. 95% purity; RP-HPLC (10→60%) *R*ₜ=12.6; MS (ESI) calcd for C₆₂H₈₂N₁₄O₁₈ 1310.59; found 656.9 [(M+2H)/2]⁺, 667.9 [(M+H+Na)/2]⁺, 1311.8 [M+H]⁺, 1333.6 [M+Na]⁺, 1349.5 [M+K]⁺.

***Tert*-butyl 2-(4-acetylphenyl)acetate (6).** To a suspension of *tert*-butyl 2-bromoacetate (6.80 mL, 46.4 mmol, 1.0 equiv), Pd(OAc)₂ (336 mg, 1.50 mmol, 3 mol%), P(*o*-tolyl)₃ (1.36 g, 4.46 mmol, 10 mol%) and K₂CO₃ (34.6 g, 0.25 mmol, 5.4 equiv) in THF (160 mL) under argon was added a solution of 4-acetylphenylboronic acid (**4**) (9.84 g, 60.0 mmol, 1.3 equiv) in THF/H₂O (160 mL/2.2 mL) drop wise over 1 h. After stirring for 18 h at room temperature, the mixture was filtered and the solvent removed under reduced pressure. The residue was taken up in EtOAc (250 mL) and subsequently washed with saturated aqueous NH₄Cl (150 mL), saturated aqueous NaHCO₃ (150 mL) and brine. After drying over MgSO₄, the organic layer was filtered through silica gel and concentrated. Flash chromatography on silica gel (EtOAc/hexane 1:8) yielded **6** (6.78 g, 63%) as a pale yellow solid. *R*_{f}=0.27 (EtOAc/hexane, 1:4); mp 50-52°C; ¹H NMR (360 MHz, CDCl₃) δ 7.92 (d, *J*=8.2 Hz, 2H), 7.37 (d, *J*=8.1 Hz, 2H), 3.58 (s, 2H), 2.59 (s, 3H), 1.43 (s, 9H); ¹³C NMR (90 MHz, CDCl₃) δ 197.7, 170.0, 140.1, 135.8, 129.4 (2C), 128.5 (2C), 81.2, 42.6, 28.0, 26.5; HRMS (EI) calcd for C₁₄H₁₈O₃ 234.12559; found 234.12532.

***Tert*-butyl 2-(4-acetylphenyl)-2-bromoacetate (8).** To a solution of **6** (2.84 g, 12.1 mmol, 1.0 equiv) in dry CCl₄ (250 mL) was added *N*-bromosuccinimide (2.58 g, 14.5 mmol, 1.2 equiv) and Br₂ (2 drops). The mixture was heated to reflux, illuminated with a 500 W halogen lamp for 10 min and stirred for further 50 min under reflux. After cooling to room temperature, the reaction solution was filtered, the solvent concentrated and the crude product purified by flash chromatography on silica gel (EtOAc/hexane 1:10) to give **8** (3.34 g, 88%) as a pale yellow solid. *R*_{f}=0.27 (EtOAc/hexane, 1:4); mp 54-56°C; ¹H NMR (360 MHz, CDCl₃) δ 7.93 (d, *J*=8.4 Hz, 2H), 7.62 (d, *J*=8.3 Hz, 2H), 5.27 (s, 1H), 2.59 (s, 3H), 1.45 (s, 9H); ¹³C NMR (90 MHz, CDCl₃) δ 197.2, 166.6, 141.1, 137.3, 128.8 (2C), 128.6 (2C), 83.5, 47.1, 27.6, 26.6; MS (EI) *m*/*z* (%) 314 (<1) [M(⁸¹Br)]⁺, 312 (<1) [M(⁷⁹Br)]⁺, 241 (10) [M(⁸¹Br)-O*t*Bu]⁺, 239 (8) [M(⁷⁹Br)-O*t*Bu]⁺; HRMS (EI) calcd for C₁₀H₈⁸¹BrO₂ [M(⁸¹Br)-O*t*Bu] 240.96872; found 240.96833, HRMS (EI) calcd for C₁₀H₈⁷⁹BrO₂ [M(⁷⁹Br)-O*t*Bu] 238.97076; found 238.97074.

***(R*/*S)-Tert*-butyl 2-[1-(1,4,7,10-tetraazacyclododecane)]-2-(4-acetylphenyl)acetate** (10). To a suspension of 1,4,7,10-tetraazacyclododecane (331 mg, 1.92 mmol, 1.2 equiv) and K₂CO₃ (552 mg, 3.99 mmol, 2.5 equiv) in DMF (60 mL) at room temperature was added a solution of **8** (500 mg, 1.60 mmol, 1.0 equiv) in DMF (50 mL) drop wise over 10 h. The mixture was filtered and concentrated under reduced pressure. Flash chromatography on silica gel (gradient MeOH/CHCl3 1:7→7:1, 1% NEt₃) yielded **10** (488 mg, 75%) as a pale yellow solid. *R*_{f}=0.10 (MeOH/CHCl₃, 3:1, 1% NEt₃); mp 33-36°C; ¹H NMR (360 MHz, CDCl₃) δ 7.91 (d, *J*=8.4 Hz, 2H), 7.43 (d, *J*=8.2 Hz, 2H), 4.62 (s, 1H), 2.90-2.67 (m, 11H), 2.64-2.44 (m, 10H), 1.47 (s, 9H); ¹³C NMR (90 MHz, CDCl₃) δ 197.4, 170.7, 142.3, 136.4, 129.3, 128.3, 81.9, 67.8, 49.3, 47.7, 45.9, 45.8, 28.1, 26.5; HRMS (EI) calcd for C₂₂H₃₆N₄O₃ 404.27875; found 404.27951.

**(*R*/*S*)-*Tert*-butyl 2-[1-(1,4,7,10-tetraazacyclododecane)-4,7,10-tris(*tert-*butylacetate)]-2-(4-acetylphenyl)acetate (11).** To a suspension of **10** (700 mg, 1.73 mmol, 1.0 equiv) and K₂CO₃ (1.08 mg, 7.79 mmol, 4.5 equiv) in DMF (50 mL) at room temperature was added a solution of *tert*-butyl 2-bromoacetate (0.84 mL, 5.71 mmol, 3.3 equiv) in DMF (20 mL) over 30 min. After stirring for 4 h, the mixture was filtrated and concentrated under reduced pressure. Flash chromatography on silica gel (MeOH/CHCl₃ 9:1, 1% TEA) yielded **11** (930 mg, 72%) as a pale yellow solid. 99% purity; RP-HPLC (10→100%) *R*ₜ=22.0; *R*_{f}=0.83 (MeOH/CHCl₃, 3:1, 1% NEt₃); mp 70-71°C; ¹H NMR (360 MHz, CDCl₃) δ 7.94 (d, *J*=8.3 Hz, 2H), 7.11 (d, *J*=8.2 Hz, 2H), 4.66 (s, 1H), 3.60 (d, *J*=17.3 Hz, 1H), 3.45 (d, *J*=17.6 Hz, 1H), 3.38 (d, *J*=17.5 Hz, 1H), 3.22-3.05 (m, 3H), 2.98-2.70 (m, 6H), 2.61 (s, 3H), 2.61-2.48 (m, 2H), 2.46-2.38 (m, 1H), 2.36-2.24 (m, 2H), 2.22-2.04 (m, 5H), 1.49 (s, 9H), 1.48 (s, 9H), 1.46 (s, 9H), 1.39 (s, 9H); ¹³C NMR (90 MHz, CDCl₃) δ 197.6, 173.4, 173.1, 173.0, 172.9, 137.2, 136.6, 130.3 (2C), 128.1 (2C), 82.9, 82.2, 82.1, 82.0, 65.4, 56.0, 55.8, 55.5, 52.7, 52.4, 52.1, 48.5, 48.1, 48.0, 47.9, 44.5, 27.9 (3C), 27.8 (6C), 27.7 (3C), 26.6; MS (EI) *m*/*z* (%) 746.0 (9) [M]⁺, 645.1 (47) [M-CO₂*t*Bu]⁺; HRMS (EI) calcd for C₃₅H₅₇N₄O₇ [M-CO₂*t*Bu] 645.42273; found 645.42257.

**Methyl 2-(4-(2-(trimethylsilyl)ethynyl)phenyl)acetate (14).** To a solution of methyl (4-iodophenyl)acetate **(13)** (12.8 g, 45 mmol, 1.0 equiv), trimethylsilyl-acetylene (9.30 mL. 67.5 mmol, 1.5 equiv) and TEA (14.9 mL, 108 mmol, 2.4 equiv) in dry CH₃CN (120 mL) at 0°C was added Pd(PPh₃)₄ (3.6 g, 3.15 mmol, 0.07 equiv) and CuI (6.00 g, 31.5 mmol, 0.7 equiv). After stirring for 30 min at 0°C and 3 h at room temperature, the mixture was filtered through a short column of silica using EtOAc/hexane (1:1) as eluent. The solvent was removed under reduced pressure and the residue purified by flash chromatography on silica gel (gradient EtOAc/hexane 1:80→1:20) to yield **14** (10.3 g, 93%) as pale yellow crystals. *R*_{f}=0.27 (EtOAc/hexane, 1:10); mp 55-58°C; ¹H NMR (360 MHz, CDCl₃) δ 7.42 (d, *J*=8.4 Hz, 2H), 7.21 (d, *J*=8.5 Hz, 2H), 3.68 (s, 3H), 3.61 (s, 2H), 0.25 (s, 9H); ¹³C NMR (90 MHz, CDCl₃) δ 171.4, 134.3, 132.0 (2C), 129.1 (2C), 122.0, 104.7, 94.2, 52.0, 41.0,-0.0 (3C); HRMS (EI) calcd for C₁₄H₁₈O₂Si 246.10761; found 246.10744.

**Methyl 2-bromo-2-(4-(2-(trimethylsilyl)ethynyl)phenyl)acetate (15).** To a solution of **14** (2.07 g, 8.40 mmol, 1.0 eq) in dry THF (20 mL) at -78°C was added LDA (2M solution in THF/n-heptane/ethylbenzene, 5.04 mL, 10.1 mmol, 1.2 equiv) and the solution stirred for 1 h. After this time, a suspension of *N*-bromosuccinimide (1.79 g, 10.1 mmol, 1.2 equiv) in dry THF (20 mL) was added and the mixture warmed to room temperature over 18 h. The solvent was removed under reduced pressure, the residue suspended in CCl₄ (30 mL), filtered and evaporated. Purification by flash chromatography on silica gel (gradient EtOAc/hexane 1:80 → 1:20, 1% NEt₃) gave **15** (1.28 g, 47%, 92% related to recovered **14**). *R*_{f}=0.42 (EtOAc/hexane, 1:10); mp 82-84°C; ¹H NMR (360 MHz, CDCl₃) δ 7.47 (d, *J*=8.7 Hz, 2H), 7.44 (d, *J*=8.7 Hz, 2H), 5.32 (s, 1H), 3.77 (s, 3H), 0.25 (s, 9H); ¹³C NMR (90 MHz, CDCl₃) δ 168.3, 135.7, 132.2 (2C), 128.5 (2C), 124.2, 104.1, 95.8, 53.3, 45.8, 45.8, -0.1 (3C); HRMS (EI) calcd for C₁₄H₁₇⁸¹BrO₂Si 326.01608; found 326.01622.

**(*R*/*S*)-Methyl 2-[1-(1,4,7,10-tetraazacyclododecane)1-2-(4-(2-(trimethylsilyl)ethynyl)phenyl)acetate (16).** To a suspension of 1,4,7,10-tetraazacyclododecane (cyclen) (548 mg, 3.18 mmol, 1.2 equiv) and K₂CO₃ (439 mg, 3.18 mmol, 1.2 equiv) in DMF (60 mL) at room temperature was added a solution of **15** (862 mg, 2.65 mmol, 1.0 equiv) in DMF (50 mL) drop wise over 10 h. The mixture was filtered and concentrated under reduced pressure. Flash chromatography on silica gel (gradient MeOH/CHCl₃ 1:9→9:1, 1% NEt₃) yielded **16** (590 mg, 53%) as a pale yellow solid. *R*_{f}=0.10 (MeOH/CHCl₃, 3:1, 1% NEt₃); mp 70-75°C; ¹H NMR (500 MHz, MeOH-d₄) δ 7.49 (d, *J*=8.2 Hz, 2H), 7.30 (d, *J*=8.3 Hz, 2H), 4.90 (s, 1H), 3.78 (s, 3H), 3.21-3.01 (m, 6H), 3.01-2.82 (m, 8H), 2.71-2.62 (m, 2H), 0.23 (s, 9H); ¹³C NMR (125 MHz, MeOH-d₄) δ 174.2, 135.6, 133.2 (2C), 130.8 (2C), 124.9, 105.4, 95.9, 67.2, 53.0, 48.4 (2C), 47.0 (2C), 44.9 (2C), 44.4 (2C), -0.0 (3C); MS (ESI) calcd for C₂₂H₃₆N₄O₂Si 416.3; found 417.4 [(M+H)]⁺, 439.4 [(M+Na)]⁺.

**(*R*/*S*)-Methyl 2-[1-(1,4,7,10-tetraazacyclododecane)-4,7,10-tris(*tert*-butylacetate)]-2-(4-ethynyl)phenyl)acetate (3).** To a suspension of **16** (530 mg, 1.27 mmol, 1.0 equiv) and K₂CO₃ (634 mg, 4.57 mmol, 3.6 equiv) in DMF (50 mL) at room temperature was added a solution of *tert*-butyl 2-bromoacetate (615 µL, 4.19 mmol, 3.3 equiv) in DMF (20 mL) over 30 min. After stirring for 4 h, the mixture was filtered, concentrated under reduced pressure and the residue dissolved in THF (20 mL). Then, tetrabutylammonium fluoride (481 mg, 1.52 mmol, 1.2 eq) was added, and after stirring for 15 min, the solvent was removed and the crude product purified by flash chromatography on silica gel (MeOH/CHCl₃ 1:10, 1% NEt₃) to yield **3** (508 mg, 85%) as a pale yellow solid. 99% purity; RP-HPLC (10→100%) *R*ₜ=20.0; *R*_{f}=0.28 (MeOH/CHCl₃, 1:9, 1% NEt₃); mp 63-68°C; ¹H NMR (500 MHz, MeOH-d₄) δ 7.48 (d, *J*=8.2 Hz, 2H), 7.20 (d, *J*=8.0 Hz, 2H), 4.83 (s, 1H), 3.75 (d, *J*=17.2 Hz, 1H), 3.74 (s, 3H), 3.55 (s, 1H), 3.54 (d, *J*=17.5 Hz, 1H), 3.51 (d, *J*=17.6 Hz, 1H), 3.26-3.21 (m, 1H), 3.18-3.05 (m, 4H), 2.99-2.92 (m, 3H), 2.89 (d, *J*=17.6 Hz, 1H), 2.87 (d, *J*=17.6 Hz, 1H), 2.74-2.63 (m, 2H), 2.33 (d, *J*=11.5 Hz, 1H), 2.28-2.12 (m, 4H), 2.12-2.05 (m, 2H), 1.54 (s, 9H), 1.52 (s, 18H); ¹³C NMR (125 MHz, MeOH-d4) δ 176.3, 175.4, 175.1, 174.7, 134.1, 132.8, 131.7, 123.8, 83.8, 83.5, 83.1, 79.7, 66.4, 59.61, 59.59, 59.57, 57.1, 56.8, 56.7, 54.2, 53.9, 53.7, 53.2, 45.9, 28.5, 28.4, 28.3, 24.8; HRMS (EI) calcd for C₃₇H₅₈N₄O₈ 686.42546; found 686.42532.

**Chemoselective oxime ligation. Synthesis of DOTA-Tyr³-octreotate derivative 19. 1** (3.3 mg, 4.5 µmol, 1.0 equiv) was deprotected in 10N aqueous HCl in dioxane (50/50, v/v; 2 mL) for 18 h after which the solvent was removed under reduced pressure. The residue was dissolved in CH₃CN/H₂O (1:1, v/v; 0.2 mL, HPLC grade) at pH 4 (TFA, HPLC grade) and **18** (6.1 mg, 4.5 µmol, 1.0 equiv) was added. After stirring for 18 h, the solvent was concentrated and the crude product was directly purified by semipreparative RP-HPLC (20→50%, 30 min) to yield **19** (6.1 mg, 73%) as a colorless powder after lyophilization. 97% purity; RP-HPLC (10→60%) *R*ₜ=18.1; MS (ESI) calcd for C₇₅H₉₉N₁₅O₂₂S₂ 1625.7; found 1626.6 [M+H]⁺, 1664.6 [M+K]⁺.

**Chemoselective 1,3-dipolar cycloaddition. ("click" chemistry). Synthesis of DOTA-Tyr³-octreotate derivative 24.** To a solution of **3** (3.1 mg, 4.1 µmol, 1.0 equiv) in THF (0.2 mL) at room temperature was added a solution of LiOH (0.33 mg, 14 µmol, 3.4 equiv) in H₂O (30 µL) and the mixture stirred for 18 h. Subsequently, H₂O (0.2 mL), peptide **25** (5.5 mg, 4.1 µmol, 1.0 equiv), 0.1M aqueous CuSO₄ (49 µL, 4.9 µmol, 1.2 equiv) and copper powder (10 mg) were added and the mixture stirred for 18 h. After this time, the copper powder was filtered off, the solvent removed under reduced pressure and the *tert*-butyl esters were cleaved by treating with a mixture of TFA/TIPS/H₂O (95:5:5, v/v; 1 mL) for 2 h. The solvent was again removed and the residue was taken up THF/H₂O (1:1, v/v, 1 mL) to precipitate the copper salts by addition of Na₂S (Na₂S * 9H₂O; 12 mg, 49 µmol, 12.0 equiv). The mixture was filtrated and the crude product directly purified by semipreparative RP-HPLC (20→50%, 30 min) to yield linear **24** (3.0 mg, 37%) as a colorless powder after lyophilization. The linear peptide was recyclized in quantitative yields by stirring in CH₃CN/H₂O/DMSO (1:1:0.1, 4 mL) for 48h. After evaporation and lyophilization from CH₃CN/H₂O (1:2, v/v, 10 mL, pH 1-3 (TFA)) **24** (3.0 mg, 37% from **25**) was isolated as a white powder. 97% purity; RP-HPLC (10→60%) *R*ₜ=16.1; MS (ESI) calcd for C₈₀H₁₀₆N₁₈O₂₂S₂ 1734.7; found 868.8 [(M+2H)/2]⁺, 1735.5 [M+H]⁺.

**⁶⁸Ga-labeling of 19.** ⁶⁸GaCl₃ was eluted with 0.1 N HCl from an in-house "OBNINSK" ⁶⁸Ge/⁶⁸Ga-generator (1.48 GBq; Chemotrade Chemiehandelsgesellschaft mbH, Leipzig, Germany). For ⁶⁸Ga-labeling, the generator eluate was first concentrated in vacuo to a final volume of app. 200 µL. Although not usually carried out, this concentration step was necessary to be able to fully exploit the comparatively low ⁶⁸Ga-activity provided by the old generator used for this experimental study. The ⁶⁸Ga-activity was then diluted with 230 µL 0.1 N NaOAc (pH = 4.5) and buffered to pH 3.5 by the addition of 80 µL 1N NaOH. After adding 2.2 µL of a 0.7 mM peptide **19** stock solution (corresponding to 1.5 nmol (3 µg) of peptide), the reaction mixture was heated to 95°C for 15 min. After cooling to room temperature, the reaction mixture was diluted with 2 mL of water. To remove unreacted ⁶⁸Ga-activity, the labeled peptide [⁶⁸Ga]**19** was immobilized on a Sep-Pak C-18 cartridge, washed with 10 mL of water and eluted with 2 mL of ethanol. To obtain an ethanol-free solution for injection into mice, the solvent was then evaporated in vacuo and the product was redissolved in 2 mL of PBS to a final activity concentration of 38 µCi/100 µL. Analytical HPLC for quality control was performed on a Nucleosil 100 C18 (5 µm, 125 x 4.0 mm) column using a Sykam gradient HPLC System (Sykam GmbH, Fürstenfeldbruck, Germany) and an UVIS 200 photometer (Linear^{™} Instrument Cooperation, Reno, USA). For radioacitvity measurement, the outlet of the UV-photometer was connected to a Na(Tl) well-type scintillation counter Ace Mate^{™} 925-Scint (EG&G Ortec, München, Germany). The eluents used were H₂O (0.1% TFA; solvent A) and CH₃CN (0.1 % TFA; solvent B), and the gradient was: 0-2 min, 0% B; 2-9 min, 0-40% B; 9-15 min, 40% B. Peptides were eluted at a constant flow of 1 mL/min. The UV detection wavelength was 220 nm. *R*ₜ ([⁶⁸Ga]**19**)=15.3 min; K' = 8.13.

**Animal experiments.** Due to its high sst₂-somatostatin receptor expression, the rat pancreatic tumor cell line AR42J was used as a tumor model.⁵⁵ To establish tumor growth, cells were detached from the surface of the culture flasks using 1 mM EDTA in PBS, centrifuged and re-suspended in serum-free culture medium (RPMI-1640, Biochrom, Berlin, Germany). Concentration of the cell suspension was 3.7 x 10⁶ cells/100µL serum. Nude mice (female, 6-8 weeks) were injected 100 µL of the cell suspension subcutaneously into the flank. Ten days after tumor transplantation all mice showed solid palpable tumor masses (tumor weight 0.7-1.4 g) and were used for the experiments.

For biodistribution studies, mice were intravenously injected 38 µCi [⁶⁸Ga]**19** (corresponding to 0.15 µg of peptide) in 100 µL PBS into the tail vein. Non-specific tissue accumulation of the radioligand was determined by coinjection of an excess of cold competitor (20 µg Tyr³-octreotide / mouse). At different time points after radioligand injection (30 and 60 min p.i.) mice (n = 5 per time point; n = 3 for blocking study at 60 min p.i.) were sacrificed and dissected. The organs of interest were removed, weighed and counted in a γ-counter (Wallach, Turku, Finland). Data are expressed as percent injected dose per gram tissue (%iD/g).

### Results

**Development of carbonyl-substituted DOTA derivatives for chemoselective attachment to aminooxy-functionalized peptides via oxime ligation.** Planning our synthetic strategy, we decided to attach the keto functionality to the DOTA moiety, as the aminooxy group can be easily implemented in peptides as a *tert*-butyloxycarbonyl-protected aminooxy-functionalized amino acid building block. As keto functionality, a methylketone was preferred over an aldehyde due to its significant higher stability. This procedure avoids additional protection and deprotection steps, which would have been essential when working with an aldehyde, and makes the final compound easily storable for longer periods. Furthermore, our idea was to widen the scope of potential applications of our new DOTA derivatives. While in the past the main focus was on derivatives allowing selective formation of monoconjugates, the development of novel DOTA derivatives offering two different functionalities which could be converted selectively would open new opportunities, for example, the selective synthesis of homo- and heterooligomers⁴¹ or the attachment of additional groups like sugars, which has been shown to result in improved pharmacokinetics.⁴² Therefore we developed two different protecting group strategies: i) an orthogonal protection with one base labile protecting group, which enables the selective deprotection and derivatization of one carboxylic group and ii) a complete protection with acid labile groups offering a one step deprotection where only a monoconjugation is desired.

Our synthesis started with 4-acetylphenylboronic acid (**4**) which was reacted with methyl and *tert*-butyl 2-bromoacetate, respectively, in a Suzuki-type cross coupling reaction to form the corresponding 2-(4-acetylphenyl) substituted acetates **5** and **6** in good yields (Scheme 1).⁴³

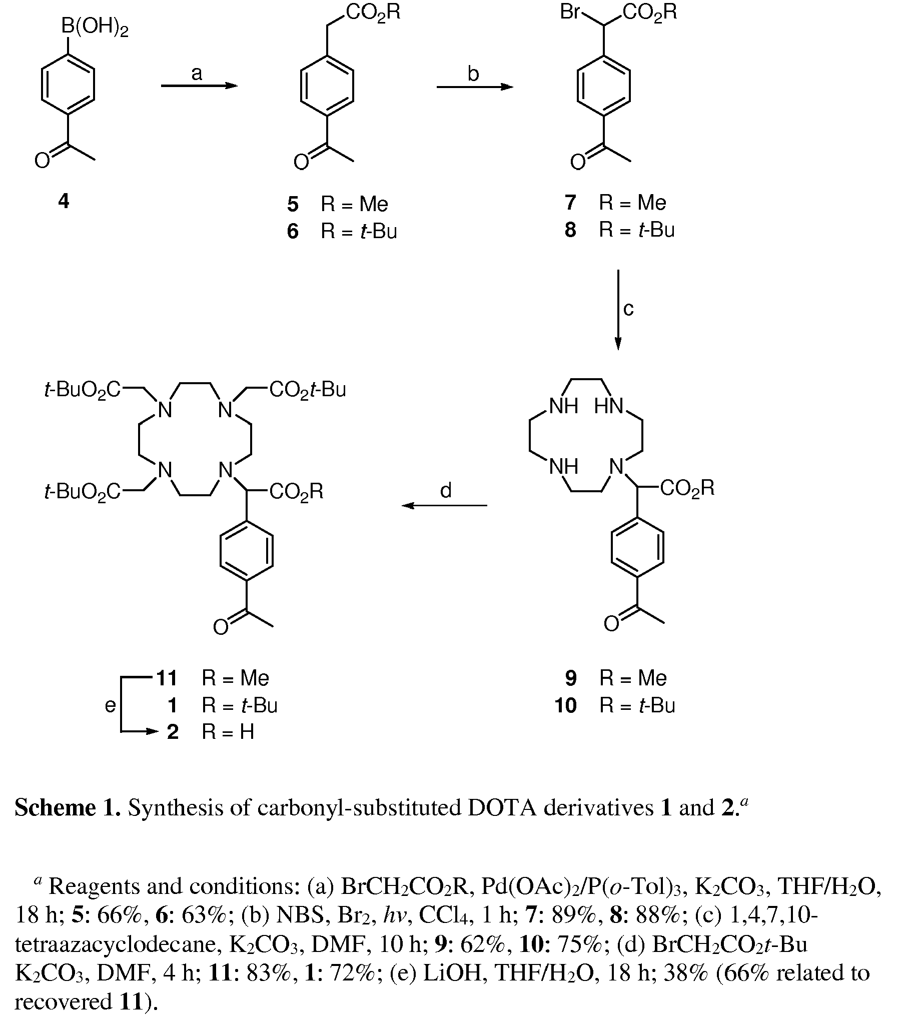

The α-bromination under radical conditions using N-bromosuccinimide in presence of catalytic amounts of bromine and initiation by illumination with light gave the α-bromoesters **7** and **8** in high yields (89% and 88%, respectively). The latter compounds were slowly added to a solution of cyclen in DMF in the presence of K₂CO₃ to afford the corresponding monoalkylated cyclen-adducts **9** and **10** in 62% and 75% yield. Subsequent peralkylation with *tert-butyl* 2-bromoacetate then gave the tetraesters **11** and **1** (83% and 72% yield, respectively). To our surprise, during saponification of the methyl ester **11** using LiOH partial deprotection of the *tert-butyl* ester was observed. However, as this side-reaction proceeds slower than the primary reaction, the desired free acid **2** could be obtained in good yield if the reaction was stopped at about 50% conversion to prevent the further cleavage of the product. In this manner **2** was obtained in 38% yield (66% based on recovered **11**) after purification by HPLC. Attempted saponification using LiI lead to unseparable mixtures of several products and when using carbonate no saponification occurred.

**Development of alkyne-substituted DOTA derivatives for chemoselective attachment to azido functionalized peptides via the Huisgen 1,3-dipolar cycloaddition.** In the design of an appropriate DOTA derivative allowing attachment to peptides via the Huisgen cycloaddition,^{18,19} we decided to attach the alkyne functionality to the DOTA derivative, as a result of the availability of azido functionalized peptides, *e.g.* by introduction of azido acids⁴⁴⁻⁴⁶ or by diazo-transfer on solid phase.⁴⁷ After our positive experiences in the synthesis of the carbonyl-substituted derivatives, we again chose 2-bromo-2-phenylacetic acid as the core residue for the implementation of an alkyne, enabling an analogous synthetic route as described above.

Commercially available 4-iodophenylacetic acid (**12**) was first protected as a methyl ester **13** (93% yield) followed by Sonogashira coupling with trimethylsilyl-acetylene using Pd(PPh₃)₄/CuI as a catalyst affored 4-alkynylphenyl acetate **14** in 93% yield (Scheme 2).

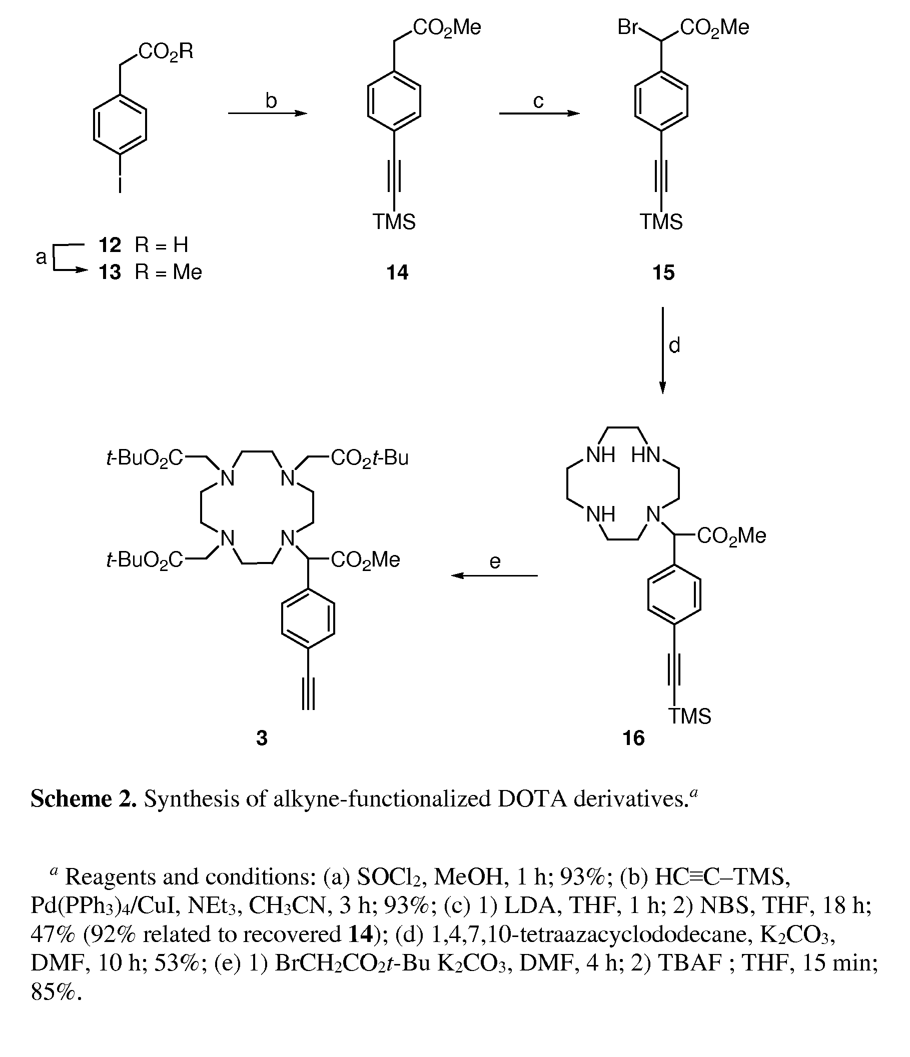

Unfortunately, the α-bromination of **14** under radicalic conditions as described above for **7** and **8** failed with both, NBS and Br₂/*hv* as initiators. This is most likely due to side reactions caused by the presence of a triple bond. Therefore, we circumvented this problem by introducing the bromide in an electrophilic manner. For this purpose, we transformed ester **14** into the boron enolate in an analogous manner as described by Evans *et al*.⁴⁸ treating subsequently with LDA and Bu₂BOTf and then adding NBS as electrophilic brominating reagent. However, yields for the α-bromo ester **15** were rather poor (39%). In an attempt to further optimize the reaction we found that the conversion proceeds very clean if the lithium enolate of **14**, obtained by deprotonation with LDA, was directly reacted with NBS. In this manner, **15** could be obtained in 47% yield together with unreacted **14** which was recovered in 49% yield. This result is explained by the much higher acidity of the α-bromo ester **15** to **14** which leads to a rapid deprotonation of the formed product with one equivalent of the lithium enolate of **14** during the reaction. We were not able to further increase the yield even if the enolate was added to a great excess of NBS. However, based on recovered starting material **14**, the yield was almost quantitative. The monoalkylation of cyclen with the α-bromo ester **15** was performed in an analogous way as described above to give **16** in 53% yield. Subsequent peralkylation with *tert*-butyl 2-bromoacetate and cleavage of the TMS protecting group using TBAF then gave the tetraester **3** (85% yield over two steps). As described in the synthesis of **2** we also tried to saponify the methyl ester in **3** selectively. But to our great surprise, in presence of one equivalent of LiOH, cleavage of a *tert*-butyl ester in **3** proceeded even faster and we found the corresponding compound with one of the *tert*-butyl and the methyl ester cleaved as the major product. The desired product among regioisomers without one *tert-butyl* group could only be detected in minor amounts.

**Chemoselecitve synthesis of the DOTA-Tyr³-octreotate conjugate 19 via oxime ligation using the DOTA ketone derivative 1.** With these novel functionalized chelators in hand we scrutinized chemoselective attachment to *N*-terminal aminooxy as well as alkyne functionalized somatostatin analogues based on Tyr³-octreotate.

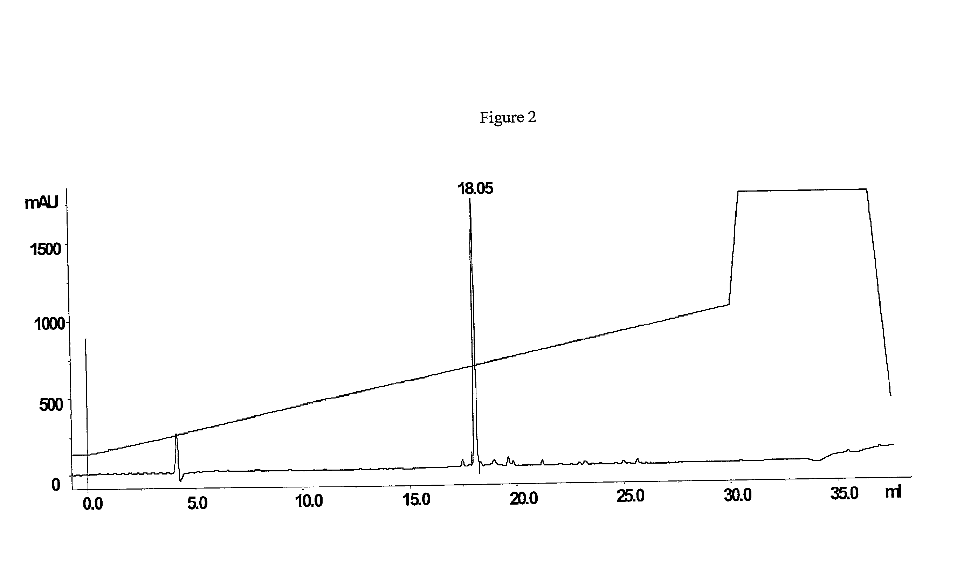

In our initial experiments, the *tert*-butyl protected DOTA ketone **1** was directly used for the oxime ligation, but although the reaction proceeded very cleanly the oxime bond of the resulting conjugate was not stable under the strong acidic conditions which were required for complete deprotection of the *tert-butyl* groups. Therefore we had to deprotect **1** *in situ* prior to the ligation. Using relatively mild methods (formic acid⁴⁹ or 50% TFA/H₂O) deprotection failed and applying a TFA/TIPS/H₂O (95/2.5/2.5, v/v/v) mixture⁵⁰ - a standard deprotection mixture used in Fmoc peptide chemistry - led to the reduction of the ketone in **1**. A quantitative cleavage was realized when treating with 10N aq. HCl in dioxane (50/50, v/v) (Scheme 3). The resulting deprotected chelator **17** was then reacted with equimolar amounts of aminooxy-functionalized Tyr³-octreotate **18** in an CH₃CN/H₂O mixture at pH 4 (TFA) to give the desired conjugate **19** (Scheme 3) with 85% purity based on HPLC analysis (Figure 2). It should be stressed that any reaction where free aminooxy functionalities occur, demand high solvent purities (HPLC grade) in order to prevent side reactions with carbonyl functionalized impurities. The final product was further purified by preparative HPLC to give **19** in 73% yield and 97% purity.

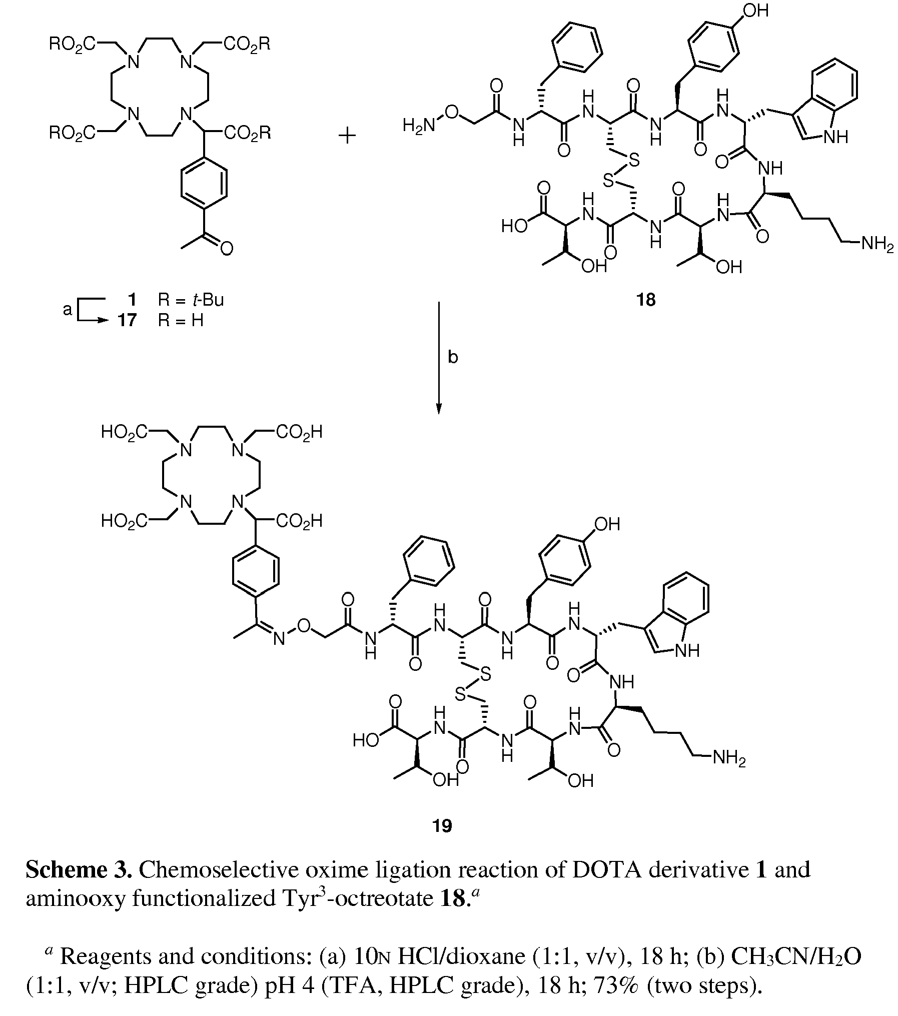

**Chemoselecitve synthesis of the DOTA-Tyr³-octreotate conjugate 24 via the Huisgen 1,3-dipolar cycloaddition using DOTA alkyne derivative 3.** With respect to the azide functionalization of Tyr³-octreotate, we chose a simple *N*-terminal elongation with 3-(3-azidopropylcarbamoyl)propanoic acid **21** on solid support. Since, in several biological active peptides it is of great importance to use a spacer between BFCA and the targeting moiety and based on earlier experience in our group **21** was the linker of choice. The two-step synthesis of **21** starting from 1-bromo-3-aminopropane is cheap, high yielding, easy to scale up and does not require any chromatography (Scheme 4).

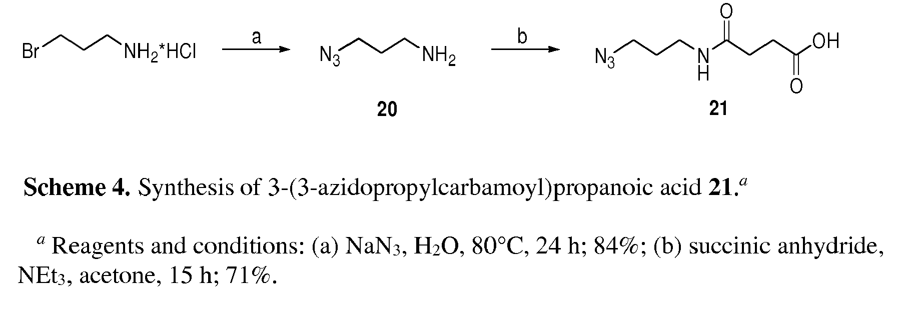

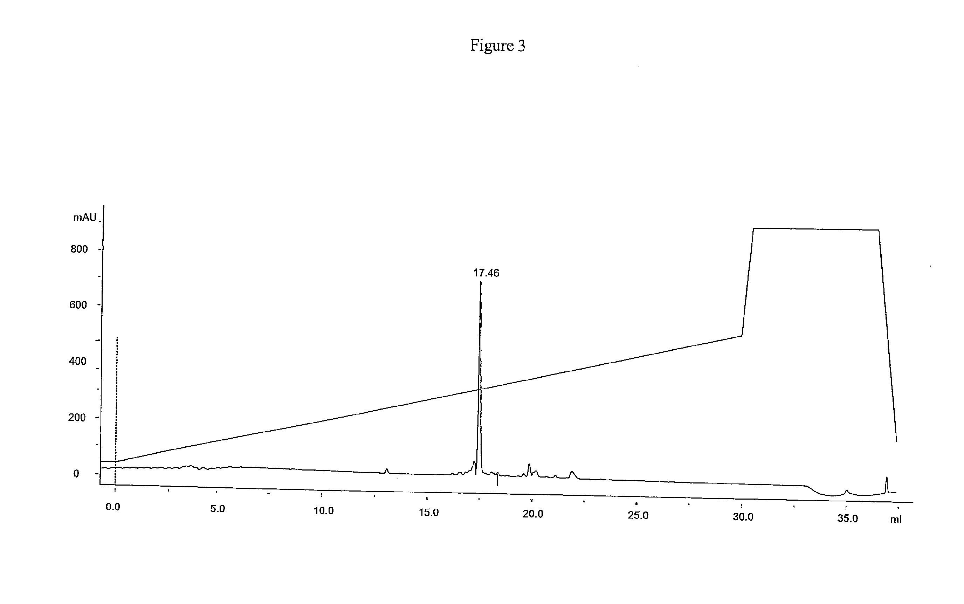

Prior to the cycloaddition methyl ester **3** was saponified *in situ* (as described above, this step goes along with the cleavage of *tert*-butyl esters) and the mixture of the resulting free acids **22** was reacted with the azido functionalized Tyr³-octreotate **23** in a THF/H₂O mixture using Cu/CuSO₄ as a catalyst system (Scheme 5). The crude product mixture then was directly deprotected using a TFA/TIPS/H₂O (95/2.5/2.5, v/v/v) mixture⁵⁰ to afford the crude product **24** (Scheme 5) with good purity (69% based on HPLC analysis, Figure 3). However, ESI mass spectroscopy showed that **24** was obtained as a highly stable copper complex which even proved to be stable during HPLC purification with acidic eluents. Nevertheless the copper salts could be easily removed by precipitation with sodium sulfide. As a consequence of this step an opening of the intramolecular disulfide bridge resulted and the peptide had to be recyclized after HPLC purification. Cyclization was achieved in quantitative yields to afford the desired conjugate **24** in 37% yield from **23** and 97% purity.

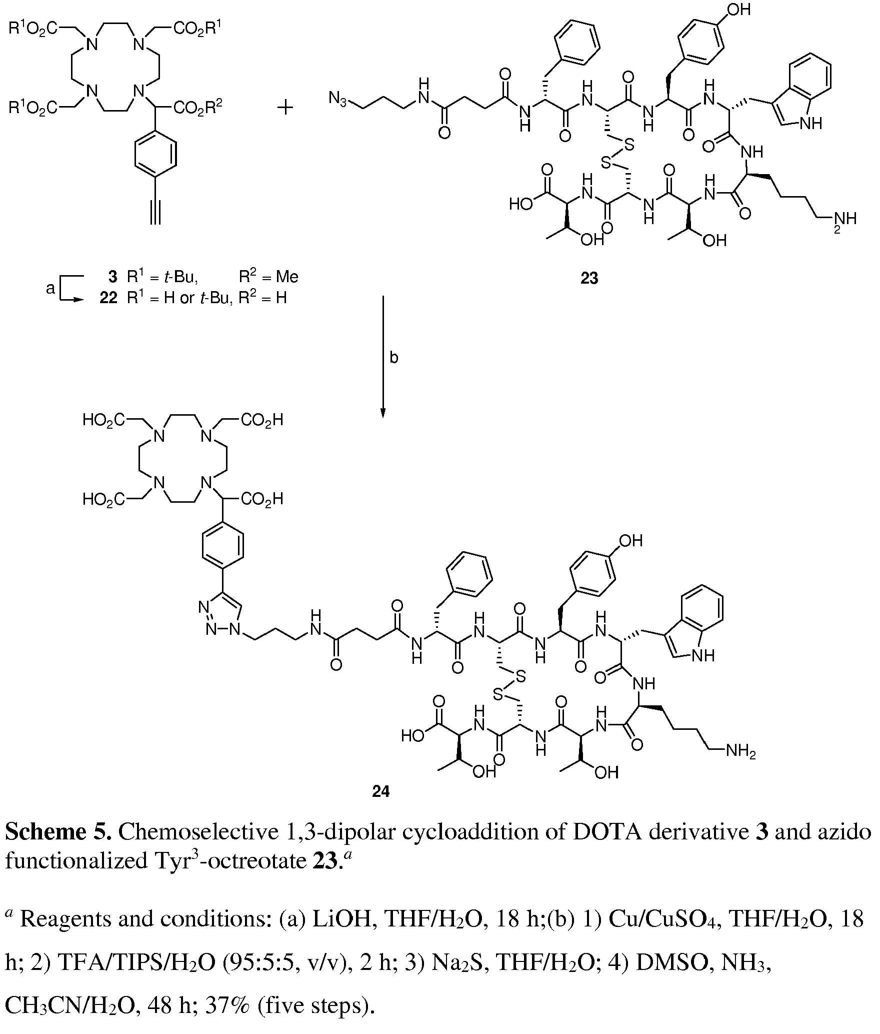

In order to check our procedure, we repeated the reaction with the azido functionalized non-cysteine containing sample peptide 3-(3-azidopropylcarbamoyl)propanoyl-Tyr-Glu-Trp-Lys (**25**, see supporting information). In an analogous manner the cycloaddition resulted in the formation of a copper complexed product, however in this instance, the metal ions could be removed without side reactions. Deprotection of the *tert*-butyl ester gave the crude product with 76% purity based on HPLC analysis. After HPLC purification the corresponding conjugate **26** was obtained in 51 % yield.

**Radiolabeling.** Usually, the ⁶⁸Ge/⁶⁸Ga-generator eluate is used directly for peptide labeling without further processing. In this study, however, the eluate was evaporated to dryness, and the ⁶⁸Ga-activity was then re-dissolved in a small volume of reaction buffer (0.1 N NaOAc, pH 3.5), which was then used for the radiometallation reaction. Although the generator was almost exhausted, this procedure allowed to reduce the amount of peptide precursor needed for efficient radiometal incorporation and thus, led to a comparably high specific activity of [⁶⁸Ga]**19** (570 Ci/mmol at the time point of injection into mice). [⁶⁸Ga]**19** was obtained in 55.7% radiochemical yield. The radiochemical purity of [⁶⁸Ga]**19** was 91.4%.

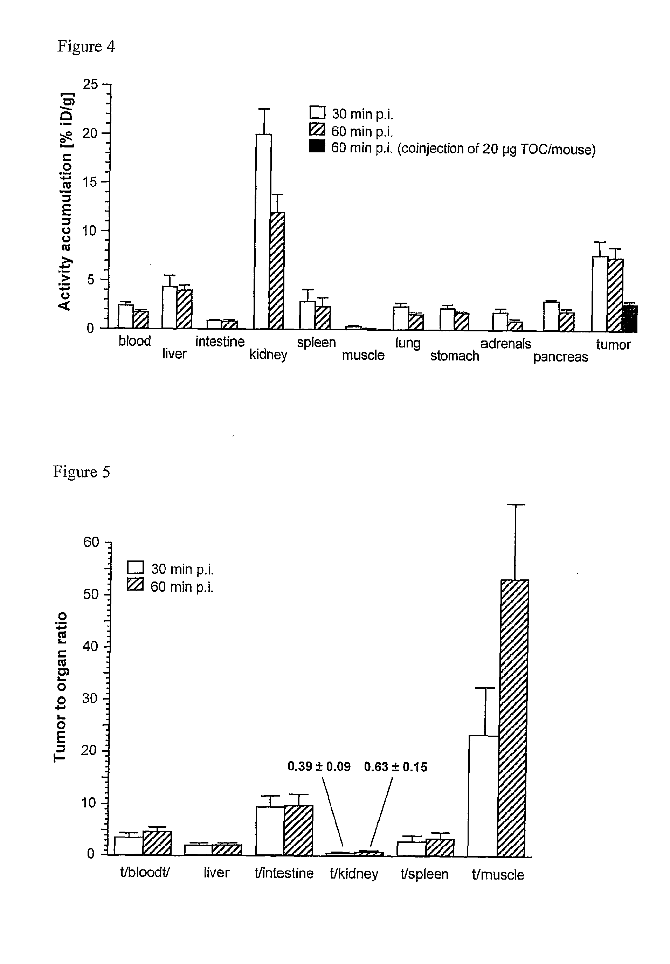

**Biodistribution studies.** The biodistribution data for [⁶⁸Ga]**19** in AR42J tumor bearing nude mice 30 and 60 min p.i. are displayed in Figure 4. At both time points, blood concentration of the radioligand was comparably high (2.37 ± 0.35 and 1.71 ± 0.17 %iD/g at 30 and 60 min p.i., respectively). While kidney accumulation rapidly decreased within the observation period (20.0 ± 2.4 to 11.9 ± 1.9 %iD/g), indicating renal clearance of [⁶⁸Ga]**19**, the tracer accumulation in the other excretion organs, i.e. liver and intestine, decreased only slowly over time, probably due to nonspecific accumulation that is not associated with excretion. In the sst-expressing tissues, a strong divergence between the tumor and the other sst-positive organs was observed. While tracer accumulation in pancreas, adrenals and stomach significantly decreased between 30 and 60 min p.i., tumor accumulation remained almost constant within this period (7.73 ± 1.52 and 7.46 ± 1.30 at 30 and 60 min p.i., respectively). This and the overall decrease in non-specific tracer accumulation in the other organs lead to increasing tumor/organ ratios between 30 and 60 min p.i. (Figure 5). That tumor accumulation is mainly receptor mediated was demonstrated in a competition study (60 min p.i.) by coinjection of an excess of unlabeled competitor (20 µg Tyr3-octreotide/mouse). Under these conditions, tumor accumulation was reduced 2.68 ± 0.36 % iD/g.

### Discussion

DOTA (1,4,7,10-tetrakis(carboxmethyl)-1,4,7,10-tetraazacyclododecane) and its derivatives emerged as an important class of chelators for imaging technologies in medicine due to their ability to form very stable complexes with a variety of di- and trivalent metal ions. For the convenient synthesis of chelator conjugated targeting biomolecules, different suitable prochelators bearing orthogonally protected carboxy-groups have been described. However, so far there is only one report on the synthesis prochelators enabling connections other than through amine or carboxyl functionalities within the targeting molecule.¹⁵ Our approach in developing BFCAs which allow chemoselective attachment via oxim ligation or click chemistry resulted in the synthesis of novel chelators **1**, **2**, and **3**. The first critical step in the synthesis is the monoalkylation of cyclen. In the literature, there are several examples of similar monoalkylations where 2⁷ or 5⁸ equivalents of cyclen are used and where the quantitative yields are calculated based on the amount alkylating agents. There are also attempts to use metals as protecting groups.⁵¹ As a result of cyclen being the most expensive reagent in the sequence, in our synthesis we used almost equimolar amounts of the alkylating agent and obtained satisfying yields between 53, 62 and 75% for compounds **16**, **9** and **10** respectively based on cyclen. Although the crude product could be directly further alkylated, purification of this intermediate was carried out via flash chromatography at this stage as it was easier to separate it from higher alkylated products and unreacted cyclen rather than separation at a later stage, where the resulting compounds only differ in the nature of the alkylated acetic ester residue. It should be also noted that in general separation of cyclen derivatives on silica is sometimes quite laborious due to diffuse bands and that purification by preparative RP-HPLC is a good alternative. The syntheses of the α-bromoesters **7**, **8** and **15** were straightforward and the products were obtained in good yields.

Final saponification of the methyl esters in **3** and 11 surprisingly also led to a hydrolysis of the *tert-butyl* esters under several conditions. For **3**, it was possible to stop the reaction at about 50% conversion to obtain **2** as a single product. Unfortunately, in the case of alkyne derivative **11** the cleavage of *tert-butyl* esters proceeded even faster. Hence, we carried out the chemoselective 1,2,3 triazole formation with a mixture of free acids **22** and subsequently cleaved the remaining *tert-*butyl groups by treatment with TFA. For the click reaction reaction^{28,29,31-36} we used 1.2 equivalents of copper sulfate together with of an excess of copper metal, which leads to *in situ* generation of the catalytic active Cu^{I} species. Although there are reports⁵² where only 1 mol% of Cu^{II} salts are used, we had to use an excess of it, given the fact that one equivalent of the Cu^{II} is immediately complexed by the DOTA derivative **22.** However, in the literature there are a plethora of different procedures⁵² and for our purposes, optimization of the catalyst loading was not essential.

Using model peptide **25**, a clean conversion to the 1,2,3-triazole derivative was observed. Facing the essential precipitation with sodium sulfide to access the metal free compound followed by the required recyclization of the opened disulfide bridge in **24**, chemoselective introduction of chelators in this manner could not be recommended for targeting molecules containing disulfide bridges as is the case in Tyr³-octreotate. However, for non-cysteine containing targeting moieties our alkyne derivatized chelator offers another good alternative for attachment of BFCAs in a highly chemoselective manner as demonstrated by reaction with model peptide **25**. Very recently Lin and coworkers have demonstrated a site-specific protein modification through Cu^{I}-catalyzed 1,2,3-triazole formation.⁵³ Applying this methodology and using alkyne functionalized DOTA derivative **22** would allow site specific DOTA labelling of proteins.

The oxim ligation of our new methyl ketone functionalized chelator **17** with unprotected *N*-terminal aminooxy-functionalized Tyr³-octreotate proceeded smoothly and without by-products thereby adding another example of the chemoselective condensation of a methyl ketone and a hydroxylamine. However, the rate of the reaction of a ketone with an aminooxy group is much slower than compared with an aldehyde as we have proven in a competition experiment exposing **18** simultaneously to benzaldehyde and acetophenone, which exclusively leads to the benzaldoxime. After a four hour reaction time, using equimolar amounts of methyl ketone **17**, the conversion was about 60% and went to completion when left overnight. Hence, we have successfully shown the chemoselective attachment of a novel bench stable DOTA derivative suitable for the ligation of aminooxy-functionalized biomolecules. In comparison to the recently presented aminooxyfunctionalized DOTA-derivatives,¹⁵ our inverse approach allows the introduction of our chelator at any position in a peptide sequence, since appropriate aminooxy functionalized building blocks for SPPS are commercially available (*e.g*. Boc-Ams(Fmoc)-OH and Fmoc-Dpr(Boc-Aoa)-OH).

In a first in vivo evaluation in AR42J tumor bearing nude mice it was demonstrated, that [⁶⁸Ga]**19** showed specific and high tumor accumulation at 30 and 60 min p.i., indicating that sst-receptor affinity remains nearly unaffected by the ligation chemistry employed. Although tumor accumulation and tumor to organ ratios found for [⁶⁸Ga]**19** are not as high as those documented for the "reference" ligands [⁶⁸Ga]DOTATOC (DOTA-Tyr³-octreotide) and [⁶⁸Ga]DOTATATE (DOTA-Tyr³-octreotate) in the same tumor model,⁵⁴ radiometallated octreotate analogs based on the DOTA-coupling methodology presented in this study may also be used for in vivo sst-imaging, probably after optimization. In this context, [⁶⁸Ga]**19** may serve as a proof of principle for the general applicability of the DOTA-conjugation chemistry via oxime ligation for the synthesis of novel receptor binding radiopeptides without challenging their receptor binding ability.

### Conclusion

The novel alkyne and keto functionalized DOTA derivatives described in this article allow a facile and chemoselective conjugation with polyfunctionalized compounds. Furthermore, a bifunctional derivative comprising a free carboxyl and carbonyl moiety is reported which may be a useful tool for further derivation and synthesis of higher compounds like heterodimers. With regard to the synthesis of our new modified chelators, we have developed economical straightforward procedures avoiding complicated protection group chemistry considering the final application of the BFCA. This allows easy access to labeled compounds in few synthetic steps. Both the alkyne as well as the methyl ketone functionalized DOTA derivatives proved to react highly selectively in the corresponding conjugation reaction with appropriate *N-*terminal modified Tyr³-octreotate. Furthermore, the pharmacokinetics of [⁶⁸Ga]**19** in AR42J tumor bearing nude mice demonstrate the suitability of the chemoselective BFC-conjugation approach for the synthesis of new radiometallated peptide ligands for diagnostic and therapeutic in vivo applications.

### References

(1) Camera, L.; Kinuya, S.; Garmestani, K.; Wu, C.; Brechbiel, M. W. et al. Evaluation of the serum stability and in vivo biodistribution of CHX-DTPA and other ligands for yttrium labeling of monoclonal antibodies. J Nucl Med 1994, 35, 882-889.
(2) Fichna, J.; Janecka, A. Synthesis of target-specific radiolabeled peptides for diagnostic imaging. Bioconjug Chem 2003, 14, 3-17.
(3) Liu, S.; Edwards, D. S. Bifunctional chelators for therapeutic lanthanide radiopharmaceuticals. Bioconjug Chem 2001, 12, 7-34.
(4) Heppeler, A.; Froidevaux, S.; Eberle, A. N.; Maecke, H. R. Receptor targeting for tumor localisation and therapy with radiopeptides. Current Medicinal Chemistry 2000, 7, 971-994.
(5) Milenic, D. E.; Brady, E. D.; Brechbiel, M. W. Antibody-targeted radiation cancer therapy. Nature Reviews Drug Discovery 2004, 3, 488-498.
(6) Schottelius, M.; Schwaiger, M.; Wester, H.-J. Rapid and high-yield solution-phase synthesis of DOTA-Tyr3-octreotide and DOTA-Tyr3-octreotate using unprotected DOTA. Tetrahedron Letters 2003, 44, 2393-2396.
(7) Heppeler, A.; Froidevaux, S.; Mäcke, H. R.; Jermann, E.; Behe, M. et al. Radiometal-labelled macrocyclic chelator-derivatized somatostatin analogue with superb tumour-targeting properties and potential for receptor-mediated internal radiotherapy. Chem. Eur. J. 1999, 5, 1974-1981.
(8) Anelli, P. L.; Lattuada, L.; Gabellini, M.; Recanati, P. DOTA tris(phenylmethyl) ester: a new useful synthon for the synthesis of DOTA monoamides containing acid-labile bonds. Bioconjug Chem 2001, 12, 1081-1084.
(9) McMurry, T. J.; Brechbiel, M.; Kumar, K.; Gansow, O. A. Convenient synthesis of bifunctional tetraaza macrocycles. Bioconjug Chem 1992, 3, 108-117.
(10) Eisenwiener, K. P.; Powell, P.; Mäcke, H. R. A convenient synthesis of novel bifunctional prochelators for coupling to bioactive peptides for radiometal labelling. Bioorg Med Chem Lett 2000, 10, 2133-2135.
(11) De Leon-Rodriguez, L. M.; Kovacs, Z.; Dieckmann, G. R.; Sherry, A. D. Solid-phase synthesis of DOTA-peptides. Chemistry 2004, 10, 1149-1155.
(12) Peterson, J. J.; Pak, R. H.; Meares, C. F. Total solid-phase synthesis of 1,4,7,10-tetraazacyclododecane-N,N', N",N"'-tetraacetic acid-functionalized peptides for radioimmunotherapy. Bioconjug Chem 1999, 10, 316-320.
(13) Thumshirn, G.; Hersel, U.; Kessler, H. Multimeric cyclic RGD Peptides as potential tools for tumor targeting: solid phase peptide synthesis and chemoselctive oxime ligation. Chem. Eur. J. 2003, 9, 2717-2725.
(14) Poethko, T.; Schottelius, M.; Thumshirn, G.; Hersel, U.; Herz, M. et al. Two-step methodology for high-yield routine radiohalogenation of peptides: (18)F-labeled RGD and octreotide analogs. J Nucl Med 2004, 45, 892-902.
(15) Hovinen, J. Synthesis of aminooxy-functionalized lanthanide(III) chelates for carbonyl-group conjugation. Chemistry & Biodiversity 2006, 3, 296-303.
(16) Rose, K. Facile Synthesis of Homogeneous Artificial Proteins. J. Am. Chem. Soc. 1994, 116, 30-33.
(17) Shao, J.; Tam, J. P. Unprotected Peptides as Building-Blocks for the Synthesis of Peptide Dendrimers with Oxime, Hydrazone, and Thiazolidine Linkages. J. Am. Chem. Soc. 1995, 117, 3893-3899.
(18) Huisgen, R. Kinetics and Reaction-Mechanisms - Selected Examples from the Experience of 40 Years. Pure and Applied Chemistry 1989, 61, 613-628.
(19) Huisgen, R.; Knorr, R.; Mobius, L.; Szeimies, G. 1.3-Dipolare Cycloadditionen .23. Einige Beobachtungen Zur Addition Organischer Azide an Cc-Dreifachbindungen. Chemische Berichte-Recneil 1965, 98, 4014-4021.
(20) Marceau, P.; Bure, C.; Delmas, A. F. Efficient synthesis of C-terminal modified peptide ketones for chemical ligations. Bioorg Med Chem Lett 2005, 15, 5442-5445.
(21) Tuchscherer, G.; Grell, D.; Mathieu, M.; Mutter, M. Extending the concept of template-assembled synthetic proteins. J Pept Res 1999, 54, 185-194.
(22) Boturyn, D.; Coll, J. L.; Garanger, E.; Favrot, M. C.; Dumy, P. Template assembled cyclopeptides as multimeric system for integrin targeting and endocytosis. J Am Chem Soc 2004, 126, 5730-5739.
(23) Poethko, T.; Schottelius, M.; Thumshirn, G.; Herz, M.; Haubner, R. et al. Chemoselective pre-conjugate radiohalogenation of unprotected mono- and multimeric peptides via oxime formation. Radiochimica Acta 2004, 92, 317-327.
(24) Kurth, M.; Pelegrin, A.; Rose, K.; Offord, R. E.; Pochon, S. et al. Site-Specific Conjugation of a Radioiodinated Phenethylamine Derivative to a Monoclonal-Antibody Results in Increased Radioactivity Localization in Tumor. J. Med. Chem. 1993, 36, 1255-1261.
(25) Wahl, F.; Mutter, M. Analogues of oxytocin with an oxime bridge using chemoselectively addressable building blocks. Tetrahedron Lett. 1996, 37, 6861-6864.
(26) Canne, L. E.; Ferré-D'Amaré, A. R.; Burley, S. K.; Kent, S. B. H. Total Chemical Synthesis of a Unique Transcription Factor-Related Protein: cMyc-Max. J. Am. Chem. Soc. 1995, 117, 2998-3007.
(27) Zhang, L. S.; Torgerson, T. R.; Liu, X. Y.; Timmons, S.; Colosia, A. D. et al. Preparation of functionally active cell-permeable peptides by single-step ligation of two peptide modules. Proc. Natl. Acal. Sci. U. S. A. 1998, 95, 9184-9189.
(28) Tornoe, C. W.; Christensen, C.; Meldal, M. Peptidotriazoles on solid phase: [1,2,3]-triazoles by regiospecific copper(I)-catalyzed 1,3-dipolar cycloadditions of terminal alkynes to azides. Journal of Organic Chemistry 2002, 67, 3057-3064.
(29) Kolb, H. C.; Finn, M. G.; Sharpless, K. B. Click Chemistry: Diverse Chemical Function from a Few Good Reactions. Angew Chem Int Ed Engl 2001, 40, 2004-2021.
(30) Diaz, D. D.; Rajagopal, K.; Strable, E.; Schneider, J.; Finn, M. G. "Click" chemistry in a supramolecular environment: Stabilization of organogels by copper(I)-catalyzed azide-alkyne [3+2] cycloaddition. Journal of the American Chemical Society 2006, 128, 6056-6057.
(31) Krasinski, A.; Radic, Z.; Manetsch, R.; Raushel, J.; Taylor, P. et al. In situ selection of lead compounds by click chemistry: target-guided optimization of acetylcholinesterase inhibitors. J Am Chem Soc 2005, 127, 6686-6692.
(32) Wu, P.; Feldman, A. K.; Nugent, A. K.; Hawker, C. J.; Scheel, A. et al. Efficiency and fidelity in a click-chemistry route to triazole dendrimers by the copper(i)-catalyzed ligation of azides and alkynes. Angew Chem Int Ed Engl 2004, 43, 3928-3932.
(33) Khanetskyy, B.; Dallinger, D.; Kappe, C. O. Combining Biginelli multicomponent and click chemistry: generation of 6-(1,2,3-triazol-1-yl)-dihydropyrimidone libraries. J Comb Chem 2004, 6, 884-892.
(34) Wang, Q.; Chan, T. R.; Hilgraf, R.; Fokin, V. V.; Sharpless, K. B. et al. Bioconjugation by copper(I)-catalyzed azide-alkyne [3 + 2] cycloaddition. J Am Chem Soc 2003, 125, 3192-3193.
(35) Sen Gupta, S.; Raja, K. S.; Kaltgrad, E.; Strable, E.; Finn, M. G. Virus-glycopolymer conjugates by copper(I) catalysis of atom transfer radical polymerization and azide-alkyne cycloaddition. Chem Commun (Camb) 2005, 4315-4317.
(36) Punna, S.; Kaltgrad, E.; Finn, M. G. "Clickable" agarose for affinity chromatography. Bioconjug Chem 2005, 16, 1536-1541.
(37) Van Eijck, C. H. Treatment of advanced endocrine gastroenteropancreatic tumours using radiolabelled somatostatin analogues. Br J Surg 2005, 92, 1333-1334.
(38) Weckbecker, G.; Lewis, I.; Albert, R.; Schmid, H. A.; Hoyer, D. et al. Opportunities in somatostatin research: biological, chemical and therapeutic aspects. Nat Rev Drug Discov 2003, 2, 999-1017.
(39) Froidevaux, S.; Eberle, A. N. Somatostatin analogs and radiopeptides in cancer therapy. Biopolymers 2002, 66, 161-183.
(40) Kwekkeboom, D. J.; Mueller-Brand, J.; Paganelli, G.; Anthony, L. B.; Pauwels, S. et al. Overview of results of peptide receptor radionuclide therapy with 3 radiolabeled somatostatin analogs. J Nucl Med 2005, 46 Suppl 1, 62S-66S.
(41) Wester, H. J.; Kessler, H. Molecular targeting with peptides or peptide-polymer conjugates: just a question of size? J Nucl Med 2005, 46, 1940-1945.
(42) Wester, H. J.; Schottelius, M.; Poethko, T.; Bruus-Jensen, K.; Schwaiger, M. Radiolabeled carbohydrated somatostatin analogs: a review of the current status. Cancer Biother Radiopharm 2004, 19, 231-244.
(43) Gooßen, L. J. Pd-catalyzed synthesis of arylacetic acid derivatives from boronic acids. Chem. Comm. 2001, 669-670.
(44) Soellner, M. B.; Dickson, K. A.; Nilsson, B. L.; Raines, R. T. Site-specific protein immobilization by Staudinger ligation. Jonrnal of the American Chemical Society 2003, 125, 11790-11791.
(45) Lundquist, J. T.; Pelletier, J. C. Improved solid-phase peptide synthesis method utilizing alpha-azide-protected amino acids. Organic Letters 2001, 3, 781-783.
(46) Alper, P. B.; Hung, S. C.; Wong, C. H. Metal catalyzed diazo transfer for the synthesis of azides from amines. Tetrahedron Letters 1996, 37, 6029-6032.
(47) Rijkers, D. T. S.; van Vugt, H. H. R.; Jacobs, H. J. F.; Liskamp, R. M. J. A convenient synthesis of azido peptides by post-assembly diazo transfer on the solid phase applicable to large peptides. Tetrahedron Letters 2002, 43, 3657-3660.
(48) Evans, D. A.; Britton, T. C.; Ellman, J. A.; Dorow, R. L. The Asymmetric-Synthesis of Alpha-Amino-Acids - Electrophilic Azidation of Chiral Imide Enolates, a Practical Approach to the Synthesis of (R)-Alpha-Azido and (S)-Alpha-Azido Carboxylic-Acids. Journal of the American Chemical Society 1990, 112, 4011-4030.
(49) Chandrasekaran, S.; Kluge, A. F.; Edwards, J. A. Synthesis of Substituted Beta-Lactams by Addition of Nitromethane to 6-Oxopenicillanates and 7-Oxocephalosporanates. Journal of Organic Chemistry 1977, 42, 3972-3974.
(50) Pearson, D. A.; Blanchette, M.; Baker, M. L.; Guindon, C. A. Trialkylsilanes as Scavengers for the Trifluoroacetic-Acid Deblocking of Protecting Groups in Peptide-Synthesis. Tetrahedron Letters 1989, 30, 2739-2742.
(51) Andre, J. P.; Toth, E.; Fischer, H.; Seelig, A.; Mäcke, H. R. et al. High relaxivity for monomeric Gd(DOTA)-based MRI contrast agents, thanks to micellar self-organization. Chemistry-a European Journal 1999, 5, 2977-2983.
(52) Bock, V. D.; Hiemstra, H.; van Maarseveen, J. H. Cu-I-catalyzed alkyne-azide "click" cycloadditions from a mechanistic and synthetic perspective. European Journal of Organic Chemistry 2006, 51-68.
(53) Lin, P. C.; Ueng, S. H.; Tseng, M. C.; J.L., K.; Huang, K. T. et al. Site-Specific Protein Modification through CuI-Catalyzed 1,2,3-Triazole Formation and Its Implementation in Protein Microarray Fabrication. Angew Chem Int Ed Engl 2006, 4286-4290.
(54) Froidevaux, S.; Eberle, A. N.; Christe, M.; Sumanovski, L.; Heppeler, A. et al. Neuroendocrine tumor targeting: Study of novel gallium-labeled somatostatin radiopeptides in a rat pancreatic tumor model. International Journal of Cancer 2002, 98, 930-937.
(55) Viguerie, N.; Tahiri-Jouti, N.; Esteve, J. P.; Clerc, P.; Logsdon, C. et al. Functional somatostatin receptors on a rat pancreatic acinar cell line. Am J Physiol 1988, 255, G113-120.
(56) Carboni, B.; Benalil, A.; Vaultier, M. Aliphatic Amino Azides as Key Building-Blocks for Efficient Polyamine Syntheses. Journal of Organic Chemistry 1993, 58, 3736-3741.
(57) Carpino, L. A.; Han, G. Y. 9-Fluorenylmethoxycarbonyl amino-protecting group. J. Org. Chem. 1972, 37, 3404-3409.
(58) Carpino, L. A.; Sadat-Aalaee, D.; Chao, H. G.; DeSelm, R. H. [(9-Fluorenylmethyl)oxy]carbonyl (FMOC) amino acid fluorides. Convienient new peptide coupling reagents applicable to the FMOC/tert-butyl strategy for solution and solid-phase syntheses. J. Am. Chem. Soc. 1990, 112, 9651-9652.
(59) Merrifield, B. Solid Phase Synthesis (Nobel lecture). Angew. Chem. Int. Ed. Engl. 1985, 24, 799-810.
(60) Merrifield, R. B. Solid Phase Peptide Synthesis. I. The Synthesis of a Tetrapeptide. J. Am. Chem. Soc. 1963, 85, 2149-2154.

## Claims

1. A compound of the formula: 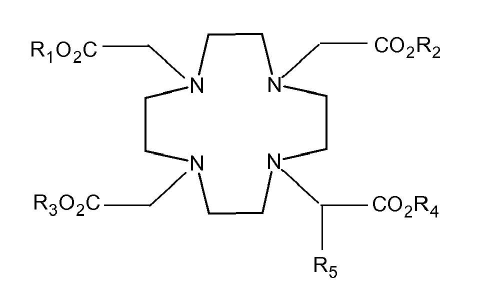 wherein R¹ is selected from H, methyl, ethyl, carboxyl protecting groups and a sugar moiety, R² and R³ are independently selected from H, methyl, ethyl and carboxyl protecting groups, R⁴ is selected from H, methyl, ethyl, a sugar moiety, and carboxyl protecting groups, and R⁵ is an aryl or heteroaryl group substituted with a keto group or a functional group suitable for participating in a cycloaddition reaction and selected from an alkyne group or an azide group, wherein the carboxyl protecting groups, when present, are selected from benzyl, fluorenylmethyl and t-butyl.

2. A compound according to claim 1 wherein the keto group is a methylketone.

3. A compound according to claim 1 wherein the alkyne group is an ethinyl group.

4. A compound according to any preceding claim wherein R⁵ is a 5-9-membered aryl or heteroaryl group comprising one or two rings.

5. A compound according to claim 4 wherein R⁵ is a 6 membered aryl or heteroaryl group.

6. A compound according to claim 5 wherein R⁵ is a phenyl group.

7. A compound according to claim 6 wherein the phenyl group is para-substituted with the keto group or the functional group suitable for participating in a cycloaddition reaction.

8. A compound according to any preceding claim wherein R¹, R² and R³ are the same or alternative carboxyl protecting groups, or wherein R¹, R² and R³ are t-butyl.

9. A compound according to any preceding claim wherein R⁴ is H or methyl.

10. A compound of the formula: 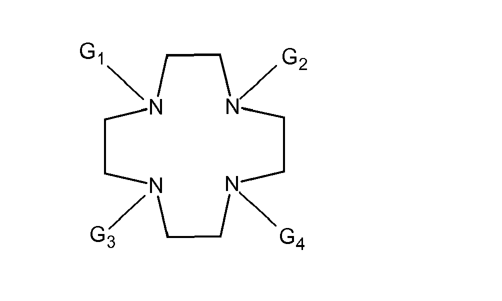 wherein from two to four of the groups G¹ to G⁴ are CH(CO₂R⁴)-R⁵ and any remaining groups G¹ to G⁴ being CH₂CO₂R¹, wherein R⁴ is selected from H, methyl, ethyl and carboxyl protecting groups, R⁵ is an aryl or heteroaryl group substituted with a keto group or a functional group suitable for participating in a cycloaddition reaction and selected from an alkyne group or an azide group, and R¹ is selected from H, methyl, ethyl, carboxyl protecting groups and a sugar moiety, wherein the carboxyl protecting groups, when present, are selected from benzyl, fluorenylmethyl and t-butyl.

11. A conjugate comprising a compound according to any preceding claim and a derivatised targeting molecule, wherein, when R⁵ is substituted with a keto group, the targeting molecule is derivatised to contain a complementary aminooxy moiety, and the compound and targeting molecule are joined by an oxime linkage and, when R⁵ is substituted with a functional group suitable for participating in a cycloaddition reaction and selected from an alkyne group or an azide group, the targeting molecule is derivatised to contain a complementary group for the cycloaddition reaction and the compound and targeting molecule are joined by means of a heterocyclic product of the cycloaddition reaction.

12. A conjugate according to claim 11, wherein the derivatised targeting molecule is a peptide.

13. A conjugate according to claim 11, wherein the compound and targeting molecule are joined by means of a 1,2,3 triazole group.

14. A chelate comprising a radionuclide complexed with a compound according to any of claims 1 to 10 or a conjugate according to claim 11.

15. A chelate according to claim 14 wherein the radionuclide is selected from Actinium-225, Bismuth-212, Bismuth-213, Lead-203, Copper-64, Copper-67, Gallium-66, Gallium-67, Gallium-68, Lutetium-177, Indium-111, Indium-113, Yttrium-86 and Yttrium-90, Dysprosium-162, Dysprosium-165, Dysprosium-167, Holmium-166, Praseodymium-142, Praseodymium-143, Promethium-149, and Terbium-49.

16. A method of synthesis of a compound according to claim 1 or claim 10, the synthesis comprising: the reaction of the di-substituted aryl or heteroaryl L¹-CH(CO₂R⁴)- R⁵-X with cyclen, wherein R⁴ and R⁵ have the same meaning as in claim 1, L¹ is a leaving group and X is a keto group or a functional group suitable for participation in a cycloaddition reaction and selected from an alkyne group or an azide group, or a protected form of such a functional group; and the alkylation of the other nitrogen atoms of the cyclen using L²CH₂CO₂R, wherein R is R¹, R² or R³ as defined in claim 1 and L² is a leaving group.

17. The method of claim 16 wherein between two and four of the nitrogen atoms of cyclen are reacted to L¹CH(CO₂R⁴)-R⁵-X, wherein R⁴ is selected from H, methyl, ethyl and carboxyl protecting groups as defined in claim 1 and R⁵ has the same meaning as in claim 1 and wherein the remaining nitrogen atoms of the cyclen are alkylated using L²CH₂CO₂R, wherein R is R² or R³ as defined in claim 1.

18. A method of synthesis of a conjugate according to claim 11, wherein the compound and the derivatised targeting molecule are reacted together prior to the optional complexation of the conjugate with a radionuclide.

19. A method according to claim 18, wherein the compound and targeting molecule are joined together by a cycloaddition reaction in the presence of a transition metal catalyst.

20. A method according to claim 19, wherein the metal catalyst is based on Cu or Rh.

21. A chelate according to claim 14, for use in therapy or diagnosis.

22. A chelate according to claim 14 for use in the diagnosis and/or treatment of hyperproliferative and/or neoplastic conditions.

23. A chelate for use according to claim 22, wherein the condition is cancer.

24. A chelate for use according to claim 23 wherein the cancer is hormone responsive.

25. A method of dechelating a metal catalyst from a bifunctional chelating agent following the metal catalysed conjugation of the bifunctional chelating agent to a targeting molecule having one or more disulfide bridges, the method comprising the removal of the metal ions using sodium sulfide, followed by treatment with NH₃ and a solvent comprising acetonitrile and water to restore the disulfide bridges.

26. A method according to claim 25, wherein the conjugation reaction involves a cycloaddition reaction.

27. A method according to claim 25 or claim 26, wherein the bifunctional chelating agent is a compound according to claim 1.

28. A method according to any of claims 25 to 27, wherein the metal catalyst is based on Cu or Rh.

29. A conjugate comprising a compound according to claim 10 and two or more targeting molecules joined to the compound through the R⁵ groups.

## Patentansprüche

1. Verbindung der Formel: 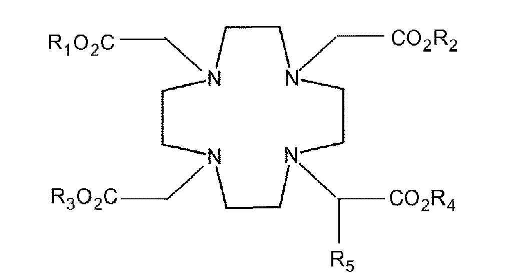 wobei R¹ ausgewählt ist aus H, Methyl, Ethyl, Carboxylschutzgruppen und einer Zuckereinheit, R² und R³ unabhängig ausgewählt sind aus H, Methyl, Ethyl und Carboxylschutzgruppen, R⁴ ausgewählt ist aus H, Methyl, Ethyl, einer Zuckereinheit und Carboxylschutzgruppen und R⁵ eine Aryl- oder Heteroarylgruppe ist, die substituiert ist mit einer Ketogruppe oder einer funktionellen Gruppe, die geeignet ist zur Teilnahme an einer Cycloadditionsreaktion und ausgewählt ist aus einer Alkingruppe oder einer Azidgruppe, wobei die Carboxylschutzgruppen, wenn sie vorhanden sind, ausgewählt sind aus Benzyl, Fluorenylmethyl und t-Butyl.

2. Verbindung nach Anspruch 1, wobei die Ketogruppe ein Methylketon ist.

3. Verbindung nach Anspruch 1, wobei die Alkingruppe eine Ethinylgruppe ist.

4. Verbindung nach einem der vorhergehenden Ansprüche, wobei R⁵ eine 5-9-gliedrige Aryl- oder Heteroarylgruppe ist, die ein oder zwei Ringe umfasst.

5. Verbindung nach Anspruch 4, wobei R⁵ eine 6-gliedrige Aryl- oder Heteroarylgruppe ist.

6. Verbindung nach Anspruch 5, wobei R⁵ eine Phenylgruppe ist.

7. Verbindung nach Anspruch 6, wobei die Phenylgruppe para-substituiert ist mit der Ketogruppe oder der funktionellen Gruppe, die geeignet ist zur Teilnahme an einer Cycloadditionsreaktion.

8. Verbindung nach einem der vorhergehenden Ansprüche, wobei R¹, R² und R³ dieselben oder alternative Carboxylschutzgruppen sind oder wobei R¹, R² und R³ t-Butyl sind.

9. Verbindung nach einem der vorhergehenden Ansprüche, wobei R⁴ gleich H oder Methyl ist.

10. Verbindung der Formel: 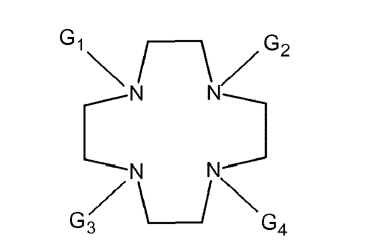 wobei von zwei bis vier der Gruppen G¹ bis G⁴ gleich CH(CO₂R⁴)-R⁵ sind und jede verbleibende Gruppe G¹ bis G⁴ gleich CH₂CO₂R¹ ist, wobei R⁴ ausgewählt ist aus H, Methyl, Ethyl und Carboxylschutzgruppen, R⁵ eine Aryl- oder Heteroarylgruppe ist, die substituiert ist mit einer Ketogruppe oder einer funktionellen Gruppe, die geeignet ist zur Teilnahme an einer Cycloadditionsreaktion und ausgewählt ist aus einer Alkingruppe oder einer Azidgruppe, und R¹ ausgewählt ist aus H, Methyl, Ethyl, Carboxylschutzgruppen und einer Zuckereinheit, wobei die Carboxylschutzgruppen, wenn sie vorhanden sind, ausgewählt sind aus Benzyl, Fluorenylmethyl und t-Butyl.

11. Konjugat umfassend eine Verbindung gemäß einem der vorhergehenden Ansprüche und ein derivatisiertes Zielmolekül, wobei, wenn R⁵ mit einer Ketogruppe substituiert ist, das Zielmolekül derivatisiert ist, um eine komplementäre Aminooxyeinheit zu enthalten, und die Verbindung und das Zielmolekül durch eine Oximverknüpfung verbunden sind, und, wenn R⁵ mit einer funktionellen Gruppe substituiert ist, die geeignet ist zur Teilnahme an einer Cycloadditionsreaktion und ausgewählt ist aus einer Alkingruppe oder einer Azidgruppe, das Zielmolekül derivatisiert ist, um eine komplementäre Gruppe für die Cycloadditionsreaktion zu enthalten, und die Verbindung und das Zielmolekül mittels eines heterocyclischen Produkts der Cycloadditionsreaktion verbunden sind.

12. Konjugat nach Anspruch 11, wobei das derivatisierte Zielmolekül ein Peptid ist.

13. Konjugat nach Anspruch 11, wobei die Verbindung und das Zielmolekül mittels einer 1,2,3-Triazolgruppe verbunden sind.

14. Chelat das ein Radionuklid umfasst, welches komplexiert ist mit einer Verbindung gemäß einem der Ansprüche 1 bis 10 oder einem Konjugat gemäß Anspruch 11.

15. Chelat nach Anspruch 14, wobei das Radionuklid ausgewählt ist aus Actinium-225, Bismuth-212, Bismuth-213, Blei-203, Kupfer-64, Kupfer-67, Gallium-66, Gallium-67, Gallium-68, Lutetium-177, Indium-111, Indium-113, Yttrium-86 und Yttrium-90, Dysprosium-162, Dysprosium-165, Dysprosium-167, Holmium-166, Praseodym-142, Praseodym-143, Promethium-149 und Terbium-49.

16. Verfahren zur Synthese einer Verbindung gemäß Anspruch 1 oder Anspruch 10, wobei die Synthese umfasst: die Umsetzung des di-substituierten Aryl oder Heteroaryl L¹-CH(CO₂R⁴)-R⁵-X mit Cyclen, wobei R⁴ und R⁵ dieselbe Bedeutung wie in Anspruch 1 besitzen, L¹ eine Abgangsgruppe ist und X eine Ketogruppe ist oder eine funktionellen Gruppe ist, die geeignet ist zur Teilnahme an einer Cycloadditionsreaktion und ausgewählt ist aus einer Alkingruppe oder einer Azidgruppe, oder eine geschützte Form einer derartigen funktionellen Gruppe ist; und die Alkylierung der anderen Stickstoffatome des Cyclen unter Verwendung von L²CH₂CO₂R, wobei R gleich R¹, R² oder R³ ist, wie in Anspruch 1 definiert, und L² eine Abgangsgruppe ist.

17. Verfahren nach Anspruch 16, wobei zwischen zwei und vier der Stickstoffatome des Cyclen umgesetzt werden mit L¹CH(CO₂R⁴)-R⁵-X, wobei R⁴ ausgewählt ist aus H, Methyl, Ethyl und Carboxylschutzgruppen, wie in Anspruch 1 definiert, und R⁵ dieselbe Bedeutung besitzt wie in Anspruch 1 und wobei die verbleibenden Stickstoffatome des Cyclen alkyliert werden unter Verwendung von L²CH₂CO₂R, wobei R gleich R² oder R³ ist, wie in Anspruch 1 definiert.

18. Verfahren zur Synthese eines Konjugats gemäß Anspruch 11, wobei die Verbindung und das derivatisierte Zielmolekül miteinander umgesetzt werden vor der optionalen Komplexierung des Konjugats mit einem Radionuklid.

19. Verfahren nach Anspruch 18, wobei die Verbindung und das Zielmolekül durch eine Cycloadditionsreaktion in Gegenwart eines Übergangsmetallkatalysators miteinander verbunden werden.

20. Verfahren nach Anspruch 19, wobei der Metallkatalysator auf Cu oder Rh basiert.

21. Chelat nach Anspruch 14 zur Verwendung in Therapie oder Diagnose.

22. Chelat nach Anspruch 14 zur Verwendung in der Diagnose und/oder Behandlung von hyperproliferativen und/oder neoplastischen Zuständen.

23. Chelat zur Verwendung nach Anspruch 22, wobei der Zustand Krebs ist.

24. Chelat zur Verwendung nach Anspruch 23, wobei der Krebs hormonresponsiv ist.

25. Verfahren zum Dechelieren eines Metallkatalysators aus einem bifunktionalen Chelatbildner nachfolgend zu der metallkatalysierten Konjugation des bifunktionalen Chelatbildners an ein Zielmolekül mit einer oder mehreren Disulfidbrücken, wobei das Verfahren das Entfernen der Metallionen unter Verwendung von Natriumsulfid, gefolgt von einer Behandlung mit NH₃ und einem Acetonitril und Wasser umfassenden Lösungsmittel zum Wiederherstellen der Disulfidbrücken umfasst.

26. Verfahren nach Anspruch 25, wobei die Konjugationsreaktion eine Cycloadditionsreaktion einschließt.

27. Verfahren nach Anspruch 25 oder Anspruch 26, wobei der bifunktionale Chelatbildner eine Verbindung gemäß Anspruch 1 ist.

28. Verfahren nach einem der Ansprüche 25 bis 27, wobei der Metallkatalysator auf Cu oder Rh basiert.

29. Konjugat umfassend eine Verbindung gemäß Anspruch 10 und zwei oder mehrere Zielmoleküle, die durch die R⁵-Gruppen an die Verbindung gebunden sind.

## Revendications

1. Composé de formule : 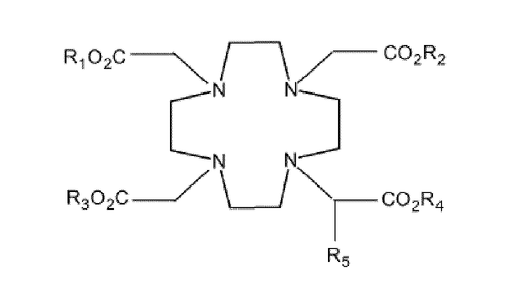 dans laquelle R¹ est choisi parmi H, des groupes méthyle, éthyle, carboxy-protecteurs et un radical de sucre, R² et R³ sont choisis indépendamment parmi H, des groupes méthyle, éthyle et carboxy-protecteurs, R⁴ est choisi parmi H, des groupes méthyle, éthyle, un radical de sucre, et des groupes carboxy-protecteurs, et R⁵ représente un groupe aryle ou hétéroaryle substitué par un groupe céto ou un groupe fonctionnel approprié pour participer à une réaction de cycloaddition et choisi parmi un groupe alcyne ou un groupe azoture, où les groupes carboxy-protecteurs, lorsqu'ils sont présents, sont choisis parmi les groupes benzyle, fluorénylméthyle et t-butyle.

2. Composé selon la revendication 1, dans lequel le groupe céto est une méthylcétone.

3. Composé selon la revendication 1, dans lequel le groupe alcyne est un groupe éthynyle.

4. Composé selon l'une quelconque des revendications précédentes, dans lequel R⁵ représente un groupe aryle ou hétéroaryle de 5 à 9 chaînons comprenant un ou deux cycles.

5. Composé selon la revendication 4, dans lequel R⁵ représente un groupe aryle ou hétéroaryle de 6 chaînons.

6. Composé selon la revendication 5, dans lequel R⁵ représente un groupe phényle.

7. Composé selon la revendication 6, dans lequel le groupe phényle est para-substitué par le groupe céto ou le groupe fonctionnel approprié pour participer à une réaction de cycloaddition.

8. Composé selon l'une quelconque des revendications précédentes, dans lequel R¹, R² et R³ représentent des groupes carboxy-protecteurs identiques ou variants, ou dans lequel R¹, R² et R³ représentent un groupe t-butyle.

9. Composé selon l'une quelconque des revendications précédentes, dans lequel R⁴ représente H ou un groupe méthyle.

10. Composé de formule : 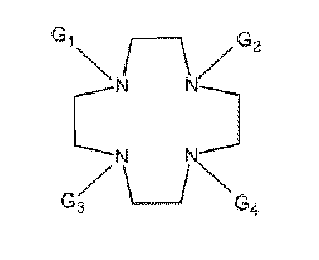 dans laquelle de deux à quatre des groupes G¹ à G⁴ représentent un groupe CH(CO₂R⁴)-R⁵ et tout groupe G¹ à G⁴ restant représentant un groupe CH₂CO₂R¹, où R⁴ est choisi parmi H, des groupes méthyle, éthyle et carboxy-protecteurs, R⁵ représente un groupe aryle ou hétéroaryle substitué par un groupe céto ou un groupe fonctionnel approprié pour participer à une réaction de cycloaddition et choisi parmi un groupe alcyne ou un groupe azoture, et R¹ est choisi parmi H, des groupes méthyle, éthyle, carboxy-protecteurs et un radical de sucre, où les groupes carboxy-protecteurs, lorsqu'ils sont présents, sont choisis parmi les groupes benzyle, fluorénylméthyle et t-butyle.

11. Conjugué comprenant un composé selon l'une quelconque des revendications précédentes et une molécule de ciblage dérivatisée, où, lorsque R⁵ est substitué par un groupe céto, la molécule de ciblage est dérivatisée pour contenir un radical amino-oxy complémentaire, et le composé et la molécule de ciblage sont joints par une liaison oxime et, lorsque R⁵ est substitué par un groupe fonctionnel approprié pour participer à une réaction de cycloaddition et choisi parmi un groupe alcyne ou un groupe azoture, la molécule de ciblage est dérivatisée pour contenir un groupe complémentaire pour la réaction de cycloaddition et le composé et la molécule de ciblage sont joints au moyen d'un produit hétérocyclique de la réaction de cycloaddition.

12. Conjugué selon la revendication 11, dans lequel la molécule de ciblage dérivatisée est un peptide.

13. Conjugué selon la revendication 11, dans lequel le composé et la molécule de ciblage sont joints au moyen d'un groupe 1,2,3-triazole.

14. Chélate comprenant un radionucléide complexé avec un composé selon l'une quelconque des revendications 1 à 10 ou un conjugué selon la revendication 11.

15. Chélate selon la revendication 14, dans lequel le radionucléide est choisi parmi Actinium 225, Bismuth 212, Bismuth 213, Plomb 203, Cuivre 64, Cuivre 67, Gallium 66, Gallium 67, Gallium 68, Lutécium 177, Indium 111, Indium 113, Yttrium86 et Yttrium90, Dysprosium 162, Dysprosium 165, Dysprosium 167, Holmium 166, Praséodyme 142, Praséodyme 143, Prométhium 149, et Terbium 49.

16. Procédé de synthèse d'un composé selon la revendication 1 ou la revendication 10, la synthèse comprenant : la réaction du groupe aryle ou hétéroaryle di-substitué L¹-CH(CO₂R⁴)-R⁵-X avec du cyclène, où R⁴ et R⁵ ont la même signification que dans la revendication 1, L¹ représente un groupe libérable et X représente un groupe céto ou un groupe fonctionnel approprié pour une participation dans une réaction de cycloaddition et choisi parmi un groupe alcyne ou un groupe azoture, ou une forme protégée d'un tel groupe fonctionnel ; et l'alkylation des autres atomes d'azote du cyclène en utilisant L²CH₂CO₂R, où R représente R¹, R² ou R³ tels que définis dans la revendication 1 et L² représente un groupe libérable.

17. Procédé selon la revendication 16, dans lequel entre deux et quatre des atomes d'azote du cyclène sont mis à réagir avec L¹-CH(CO₂R⁴)-R⁵-X, où R⁴ est choisi parmi H, des groupes méthyle, éthyle et carboxy-protecteurs tels que définis dans la revendication 1 et R⁵ a la même signification que dans la revendication 1 et où les atomes d'azote restants du cyclène sont alkylés en utilisant L²CH₂CO₂R, où R représente R² ou R³ tels que définis dans la revendication 1.

18. Procédé de synthèse d'un conjugué selon la revendication 11, dans lequel le composé et la molécule de ciblage dérivatisée sont mis à réagir ensemble avant la complexation éventuelle du conjugué avec un radionucléide.

19. Procédé selon la revendication 18, dans lequel le composé et la molécule de ciblage sont joints ensemble par une réaction de cycloaddition en présence d'un catalyseur de métal de transition.

20. Procédé selon la revendication 19, dans lequel le catalyseur de métal est à base de Cu ou Rh.

21. Chélate selon la revendication 14, pour une utilisation en thérapie ou diagnostic.

22. Chélate selon la revendication 14, pour une utilisation dans le diagnostic et/ou le traitement d'affections hyperprolifératives et/ou néoplasiques.

23. Chélate pour une utilisation selon la revendication 22, où l'affection est le cancer.

24. Chélate selon la revendication 23, où le cancer est sensible aux hormones.

25. Procédé de déchélation d'un catalyseur de métal à partir d'un agent chélateur bifonctionnel à la suite de la conjugaison catalysée par le métal de l'agent chélateur bifonctionnel à une molécule de ciblage comportant un ou plusieurs ponts disulfure, le procédé comprenant l'élimination des ions métalliques en utilisant du sulfure de sodium, suivie d'un traitement par NH₃ et un solvant comprenant de l'acétonitrile et de l'eau pour restaurer les ponts disulfure.

26. Procédé selon la revendication 25, dans lequel la réaction de conjugaison implique une réaction de cycloaddition.

27. Procédé selon la revendication 25 ou la revendication 26, dans lequel l'agent chélateur bifonctionnel est un composé selon la revendication 1.

28. Procédé selon l'une quelconque des revendications 25 à 27, dans lequel le catalyseur de métal est à base de Cu ou Rh.

29. Conjugué comprenant un composé selon la revendication 10 et deux molécules de ciblage ou plus jointes au composé par l'intermédiaire des groupes R⁵.
